# EUROPEAN PATENT APPLICATION

(11) **EP 4 741 545 A1**
(43) Date of publication of application: **13.05.2026**
(21) Application number: 24211496.5
(22) Date of filing: 07.11.2024
(51) Int. Cl.: D01D 5/253, D01D 5/34, D01F 8/06, D06N 7/00, E01C 13/08, D02G 1/00, G01N 33/36, D01F 1/10, D01F 6/04, D01F 6/46, D01F 6/60

(54) **HELICAL ARTIFICIAL TURF FIBER WITH DIFFERENT POLYMERS IN CORE AND PERIPHERY**

(71) Applicant: Polytex Sportbeläge Produktions-GmbH, 47929 Grefrath (DE)
(72) Inventor: Dr. SICK, Stephan, 76532 Baden-Baden (DE); Dr. GROCHLA, Dario, 44869 Bochum (DE)
(74) Representative: Richardt Patentanwälte PartG mbB

(57) **Abstract**

Disclosed is an artificial turf fiber (204, 502-510, 804, 902-906, 1500, 1602-1618) comprising a longitudinal cross-sectional profile, the artificial turf fiber being under internal stress that causes the artificial turf fiber, upon receiving heat treatment, to assume a helical shape, wherein the core of the fiber consists of or predominantly comprises a core polymer, and wherein the periphery of the fiber consists of or predominantly comprises a cladding polymer, wherein the core polymer and the cladding polymer are different polymer materials, and wherein the core and cladding polymers are selected such that the core polymer undergoes greater shrinkage than the cladding polymer when subjected to the heat treatment.

## Description

### FIELD OF THE INVENTION

The invention relates to the field of artificial turf, and methods of manufacturing the same.

### BACKGROUND

Artificial turf is a surface made of synthetic fibers designed to resemble natural grass. It is commonly used in sports fields, residential lawns, and commercial landscaping, offering low maintenance, durability, and the ability to withstand heavy use and varying weather conditions.

Helical artificial turf fibers offer several advantages over straight fibers in terms of durability, aesthetics, and performance: The helical structure allows the fibers to spring back more easily after being compressed, making the turf more durable in places where there is frequent foot traffic, such as sports fields or playgrounds. This resilience is critical for maintaining the appearance and functionality of artificial turf over time, as straight fibers are more likely to flatten or break down under constant pressure. Furthermore, the helical design gives artificial turf a more natural look and feel, mimicking the way natural grass blades appear at different angles. Straight fibers tend to reflect more sunlight, creating an unnatural shiny appearance. Helical fibers help diffuse light, reducing glare and providing a more matte finish, which enhances the natural look of the turf. The helical nature of these fibers also provides better grip and traction. This can be particularly important for sports fields, where players need a stable surface to run and play without slipping.

The Korean patent KR102535359B1 describes a method of manufacturing spiral or spring-shaped artificial turf yarn.

US patent US 10,760,225 B2 1 describes a system for manufacturing helix-shaped artificial turf filaments, wherein the system comprises a first and a second air drawn oven downstream of an extrusion spinneret consecutively heat artificial turf filaments to a first and then a second temperature, wherein the second temperature is higher than the first temperature.

### SUMMARY OF THE INVENTION

It is an objective to provide for an improved helical artificial turf fiber and for a method of manufacturing the same. The objectives underlying the invention are solved by the features of the independent claims. Embodiments of the invention can freely be combined with each other unless they are mutually exclusive.

In one aspect, the invention relates to an artificial turf fiber comprising a longitudinal cross-sectional profile, the artificial turf fiber being under internal stress that causes the artificial turf fiber, upon receiving heat treatment, to assume a helical shape, wherein the core of the fiber consists of or predominantly comprises a core polymer, and wherein the periphery of the fiber consists of or predominantly comprises a cladding polymer, wherein the core polymer and the cladding polymer are different polymer materials, and wherein the core and cladding polymers are selected such that the core polymer undergoes greater shrinkage than the cladding polymer when subjected to the heat treatment.

In a further aspect, the invention relates to a helical artificial turf fiber comprising a longitudinal cross-sectional profile, wherein the core of the fiber consists of or predominantly comprises a core polymer, and wherein the periphery of the fiber consists of or predominantly comprises a cladding polymer, wherein the core polymer and the cladding polymer are different polymer materials, and wherein the core and cladding polymers are selected such that the core polymer undergoes greater shrinkage than the cladding polymer when subjected to the heat treatment. Hence, the helical structure of the fiber is the result of subjecting the fiber to heat treatment, the heat treatment having caused the core polymer to shrink stronger than the cladding polymer.

For example, before the heat treatment, the core polymer and the cladding polymer may have the same length in any length unit of the fiber. However, as a result of the heat treatment, the length of the core of said sub-unit may be at least 2%, e.g., at least 5% or at least 10% shorter than the length of the periphery of said sub-unit. For example, the length of the core of the subunit may be measured as the distance of two points at the center of the fiber profile and the length of the periphery of the sub-unit may be measured as the distance of two points on the surface of the tip of one of the fiber arms.

The applicant has identified that the combination of a fiber with a longitudinal cross-sectional profile and the use of a core polymer that exhibits greater shrinkage compared to a cladding polymer at the periphery during heat treatment offers significant advantages in producing fibers with a pronounced and stable helical configuration. By carefully selecting polymers with differing shrinkage properties for the core and cladding materials, it becomes possible to precisely control the degree of helicity, as well as the number of twists per unit length of the fiber. The greater the disparity in shrinkage between the core polymer and the cladding polymer, the more substantial the differential in contraction between the core and the outer regions of the fiber. This differential leads to an increased tendency for the fiber to adopt a tightly wound helical shape with a higher number of twists. Consequently, the degree of helicity and the uniformity of the fiber's twisted structure can be finely tuned to meet specific design requirements, offering greater versatility and performance characteristics in various industrial and commercial applications.

The applicant has also identified several further features related to the precise control of a temperature gradient during post-extrusion processing, as well as the incorporation of specific additives, which can be used in managing the helicality and for enhancing the durability of the fiber, in particular for increasing the fiber's robustness against splicing. The careful selection of the direction and amplitude of the temperature gradient allows for better manipulation of the fiber's structural characteristics during the stretching phase, enabling greater control over the degree of helicality and ensuring a more consistent and stable twist formation. Additionally, the introduction of targeted additives can significantly improve the fiber's resistance to splicing, a prevalent challenge in helical fiber production. These additives not only enhance the fiber's mechanical integrity but also contribute to maintaining the helical structure under various operational conditions. By optimizing both the thermal processing parameters and the chemical composition of the fiber, the applicant has developed a method that minimizes the risk of splicing while producing robust, high-performance helical fibers suitable for a wide range of applications where mechanical stability and precise structural properties are critical.

In a further aspect, the invention relates to an artificial turf fiber comprising an anti-splicing agent. The artificial turf fiber is under internal stress that causes the artificial turf fiber, upon receiving heat treatment, to assume a helical shape.

The internal stress may be introduced, for example, during the post-processing of the artificial turf fiber after extrusion, such as by stretching and/or compressing the fiber while a temperature gradient is present in the fiber's cross-section. For example, this type of fiber may be sold as intermediary product on the market and may allow manufacturers of artificial turf to trigger the formation of the helical shape simply by heat-treating these fibers. For example, the heat-treatment step can be a step of applying a liquid backing, e.g., a liquid latex or polyurethane reaction mixture, onto a greige good (i.e., a carrier into which the artificial turf fibers have been integrated e.g. via tufting or weaving or knitting) and heating the greige good with the liquid backing in an oven to solidify or cure the liquid backing. This heating step in the oven may in addition trigger the formation of the helical shape which has been invisibly structurally imprinted in the material of the fiber e.g. by creating a temperature gradient in the fiber cross section and applying mechanical forces (stretching and/or compressing the fiber) while the temperature gradient is present.

The internal stress may be tensile and/or compressive stress and may be manifested in the fiber material in the form of stress fields. The internal stress may also be referred to as material-immanent stress, because it is also present when no external force is currently exerted on the material.

In a further aspect, the invention relates to a helical artificial turf fiber, comprising an anti-splicing agent.

For example, this helical fiber may be the product of applying a heat treatment step on the above-mentioned fiber. An example for this would be the step of heating a liquid backing in an oven in order to solidify the backing. For example, the heat treatment may comprise heating the artificial turf fiber (or the artificial turf or greige good comprising the same) to a temperature above 70°C, in particular a temperature between 70 °C and 130°C, in particular a temperature between 80°C and 90°C.

Applicant has observed that helical fibers typically have a higher risk of splicing, i.e., splitting in longitudinal direction, over time compared to straight fibers. The process of introducing stress into the material for ultimately manufacturing a helical fiber may comprise introducing tensile and/or compressive stress within the fiber material, which makes the fiber strands more prone to longitudinal splicing, reducing the longevity of twisted turf fibers. This is highly disadvantageous: firstly, the improved mechanical stability and ability of the fibers to re-erect after being trampled down relies on the structural integrity of the helical shape and of the whole fiber. If the shape is destroyed by the splicing process, the mechanical stability of helical fibers may soon become much worse than that of straight or non-helical fibers. Furthermore, the increased tendency of helical fibers to splice reduces the longevity and the evenness of appearance of the artificial turf comprising the same.

Applicant has not only observed that helical fibers have a higher tendency to splice than non-helical fibers, applicant has also identifies substances which are able to prevent or significantly slow down the splicing process in helical fibers.

By introducing the anti-splicing agent, or a combination of two or more of the anti-splicing agents into a polymer mixture used for the manufacturing of helical fibers, a mechanically more robust helical fiber with a longer lifetime and significantly less splicing damage can be provided. Applicant has observed that the anti-splicing agent may help prevent the splitting or fraying of artificial turf fibers as a result of exposure to mechanical stress, UV light, and temperature fluctuations. By inhibiting splicing, these agents extend the lifespan of the turf. The anti-splicing agent may ensure that the fibers maintain their structural integrity even in high-traffic areas, such as sports fields or playgrounds. This contributes to the overall durability of the turf, reducing the need for frequent replacements. The use of the anti-splicing agent may also help to prevent an uneven, worn-out appearance as the anti-splicing agents help retain the original appearance of the turf by keeping fibers intact and smooth.

With the use of anti-splicing agents, artificial turf fibers may maintain their performance characteristics, such as flexibility and resilience, over longer periods. This ensures that the turf continues to offer a comfortable and safe surface for its intended use, whether in residential, commercial, or sports settings.

Prior art systems and techniques which create artificial turf filaments with a helical form using water-drawn ovens have the disadvantage that the filaments are caused to take on a helical shape almost immediately, even before the manufacturing process (e.g. the tufting process and the application of a backing layer) are complete. Processing helical filaments is, however, more challenging and may result in production errors compared to the processing and tufting of straight filaments which are transformed into helical shape at an arbitrarily selectable point in time when the fibers are subjected to heat treatment.

According to embodiments, the stretching is performed completely or at least partially mechanically. This may have the advantage of having better control over the number and regularity of the helix turns compared to a purely temperature-induced stretching process.

For example, the fiber can be stretched mechanically by subjecting the fiber to tension between two sets of rollers that rotate at different speeds.

Some first rollers may rotate faster than some second rollers located upstream of the first rollers, thereby pulling and elongating the fibers. The difference in speed, referred to as the draw ratio, determines how much the fiber is stretched. A higher draw ratio results in greater elongation and alignment of polymer chains. For example, the first rollers may be located at the end of a stretching unit, also referred to as stretching chamber, and the second rollers may be located at the entry of the chamber. But the stretching may not be limited to the use of a stretching chamber, and some stretching steps may already be performed during transport of the fiber from a first processing unit, e.g., the quenching unit, to other processing units. Instead of rollers, other mechanical devices that exert a tension on the fiber can be used for stretching the fibers, e.g., air jets or spools for winding up the fibers.

In general, stretching of fibers is performed to improve the mechanical properties and dimensional stability of the fibers. The stretching process aligns the polymer chains within the fiber, increasing crystallinity and tensile strength, which ultimately enhances the durability and resilience of the artificial turf. The applicant has observed that stretching the fiber while a temperature gradient is established in the fiber's cross-section does not only improve the tensile strength but is also able to cause the fiber to adapt helical shape upon later heat treatment.

According to embodiments, the helical artificial turf fiber is a helical fiber in the strict sense and has a 3D-helical shape characterized in that the fiber winds around a (virtual) central cylinder. The fiber maintains a distance from the cylinder axis as it rotates, and advances along the axis, resulting in a three-dimensional spiral curve.

According to other embodiments, the helical artificial turf fiber is a twisted fiber that is rotated around its own axis.

In some embodiments, the twisted fiber is only rotated around its own axis and is not coiled around a central axis of a cylinder. In other embodiments, the twisted fiber is both rotated around its own axis and wound around a central axis of a cylinder, meaning it functions as both a twisted fiber and a helical fiber in the strict sense.

According to some embodiments, the post-processing of the extruded fiber, before it is subjected to the heat-treatment that causes the fiber to assume the helical shape, comprises a texturization step. In this case, the temperature treatment transforms a texturized fiber into a texturized, helical fiber. In other embodiments, the post-processing does not include a texturization step, and the fiber may be transformed from a straight 3D form (in mathematical terms: a cylinder) into a helical 3D shape. It is also possible that some of the post-processing steps involve an increase in temperature that may already introduce a slight helical shape. In this case, the heat-treatment transforms a slightly helical fiber into a helical fiber with an increased number of turns.

According to embodiments, the anti-splicing agent is or comprises a compatibilizer.

A compatibilizer as used herein is a chemical additive often used to improve the compatibility between different polymers that make up the turf's fibers or components. Artificial turf fibers are often composed of a blend of polymers (such as polyethylene and polypropylene or polyamide), which may not naturally mix well due to differences in their chemical structure. The role of a compatibilizer is to bridge these polymers, enhancing their ability to bond together, which results in improved mechanical properties and durability.

Applicant has surprisingly observed that compatibilizers may enhance the mechanical stability of helical fibers even in case these fibers are made of a single type of polymer, e.g. polyethylene. The mechanical stability may be improved even more in fibers which are based on a mixture of two or more different polymers such as polyethylene and polyamide or polyethylene and polypropylene.

According to some embodiments, the artificial turf fiber mainly (e.g., to more than 50%, in particular to more than 70%, in particular to more than 80%, consists of polyethylene. The rest may be another polymer and additives, e.g. polyamide and/or polypropylene and additives.

By enhancing the compatibility between different polymer components, the fibers become stronger and more resistant to wear.

According to some embodiments, the compatibilizer is selected from a group comprising: an anhydride modified polyethylene, a maleic acid grafted on polyethylene or polyamide; a maleic anhydride grafted on free radical initiated graft copolymer of polyethylene, SEBS, EVA, EPD, or polypropylene with an unsaturated acid or it's anhydride such as maleic acid, glycidyl methacrylate, ricinoloxazoline maleinate; a graft copolymer of SEBS with glycidyl methacrylate, a graft copolymer of EVA with mercaptoacetic acid and maleic anhydride; a graft copolymer of EPDM with maleic anhydride; Ethylene Ethyl Acrylate (EEA), Maleic Acide Anhydride grafted PE, a graft copolymer of polypropylene with maleic anhydride; a polyolefin-graftpolyamidepolyethylene or polyamide; and a polyacrylic acid type compatibilizer; and a combination of two or more of the aforementioned substances.

In particular Ethylene Ethyl Acrylate (EEA) and Maleic Acide Anhydride grafted PE have been observed to effectively protect helical fibers from splicing. The Maleic Acide Anhydride grafted PE may have a density of 0.90-0.96 g/cm3, in particular 0.93 g/cm3 (measured using he displacement method: ASTM D792) and a Melt Flow Index of 1.70-1.80, in particular 1.75 g/10 min (measured at 190°C/2.16 kg - ASTM D1238).

For example, the fiber (and the polymer mixture used for manufacturing the fiber) comprises the compatibilizer in an amount of 0.5- 5.0 weight %, in particular 0.5- 2.0 weight % of the fiber (or of the polymer mixture).

In particular, the compatibilizer can be EEA or Maleic Acid Anhydride grafted PE, and the fiber (and the polymer mixture used for manufacturing the fiber) comprises the compatibilizer in an amount of 0.5- 5.0 weight %, in particular 0.5- 1.0 weight % of the fiber (or of the polymer mixture).

According to some embodiments, the fiber comprises a different polymer material at its core compared to its peripheral regions (with respect to the fiber cross section). The polymer materials of the core and of the peripheral regions are selected such that the polymer material at the core exhibits greater shrinkage than the polymer material in the peripheral regions in response to heat treatment.

This may have the benefit of generating fibers which will acquire a strongly twisted helical shape upon receiving heat treatment: As the polymers in the core (e.g. in the core of the central bulb, e.g. in the core of a core-cladding structure) will shrink stronger than the polymer in the peripheral regions (e.g., the protrusion/wings) extending therefrom, the peripheral regions (protrusion/wings) will be longer than the core regions (central bulb, central spine) of the fibers. This creates stress within the fiber that could potentially result in irregular undulations of the wings. However, as the fiber was stretched while a temperature gradient was present, the length difference between the core and the peripheral regions/wings is resolved in a more regular 3D structure, namely a helical structure.

A "central bulb" as used herein is a visually distinct central area of the cross-section of the fiber, that forms a protrusion to at least one side, typically to the two sides of the fibers. Typically, the central bulb has the form of a circle or ellipse.

The combination of a cross-sectional temperature gradient during the stretching and a polymer at the core that is adapted to shrink more strongly in response to heat treatment is particularly suited to generate very regularly defined helical fibers comprising a strong twist around their own axis.

According to embodiments, the fiber has a longitudinal cross-sectional profile, i.e., a cross-sectional fiber profile with a maximum-length-to-maximum-thickness ratio of at least 2.0. According to some embodiments, the fiber has a cross-sectional profile with a maximum-length-to-maximum-thickness ratio of at least 3.0, or of at least 4.0, or of at least 5.0.

In particular for cross-sectional fiber profiles with a maximum-length to maximum thickness ratio of 3.0 or higher, or 4.0 or higher, or 5.0 or higher, the temperature gradient is a radial temperature gradient. This may have the advantage that a radial temperature gradient may be sufficient to induce a helical structure. For example the helical structure can be controlled such that the helical structure is that of a twisted screw, with no or only a small lateral component of rotation (this means, the fiber basically rotates only around its own axis and not around a surface of a virtual cylinder like a helix in the strict sense.

The heat-treatment may be, for example, the heat treatment that causes the artificial turf fiber change its 3D shape to form a helical artificial turf fiber.

For example, the fiber may comprise PE at its core and PA at its peripheral regions: given a certain temperature, e.g., 90°C, PE will shrink stronger than PA. According to a different embodiment, the fiber may predominantly consist of polyethylene having a lower HDPE content in its core than in its peripheral regions.

Hence, using two different polymers for the core of the fiber cross section, e.g., for the core of a central bulb, and for the periphery, e.g., the wings, can be accomplished with the help of a co-extrusion nozzle which is able to provide fiber profiles having a core-cladding structure with a clearly defined contact surface between the core and the cladding polymer.

However, there exist also means for establishing a different material composition in the core polymer in the core and the cladding polymer at the periphery regions with the help of a simple extrusion nozzle for a single polymer mixture. For example, the single extruded polymer mixture can be a three phase system comprising at least a first polymer (to be predominantly located at the center of the fiber, e.g., PE), a second polymer (the main polymer, with a lower tendency to shrink under heat treatment than the first polymer, e.g., PA) and a compatibilizer. The first polymer is added in a smaller amount, e.g., 5-30% by weight, to the polymer mixture and forms beads within the second polymer, the beads being surrounded by the compatibilizer. The fluid dynamics during the extrusion process will concentrate the beads predominantly in the core of the extruded fiber, thereby creating a different polymer composition in the core than in the periphery, wherein the core polymer located in the core has a higher share of the first, less rigid polymer (e.g. PE) than the cladding polymer located in the periphery (e.g., the wings), although there may not be a clearly defined contact surface that separates the core and the cladding polymer from each other as in the co-extrusion/core-cladding example. Rather, a continuous gradient of the core polymer at the core and the cladding polymer at the periphery is established. Hence, a three-phase system as described above may be used for creating, with an extrusion nozzle for a single polymer mixture, a fiber whose central region will shrink more strongly under heat-treatment than the wings.

In other embodiments, the three-phase polymer mixture may be used as the core polymer by a co-extrusion nozzle, wherein a polymer consisting purely of PA or PP may be used as the cladding polymer of the co-extrusion nozzle.

According to embodiments, the anti-splicing agent is or comprises a low-density polyethylene - LDPE.

The LDPE is a PE-variant with many side chains. The side-chains may create an increased entanglement of the polymer molecules in the fiber: LLDPE molecules and HDPE molecules do not have a sufficient degree of branching and have been therefore been observed to be prone to splicing (i.e., splitting in longitudinal direction). Adding LDPE will increase the entanglement of PE-molecules and thereby prevent splicing.

For example, the fiber may comprise the LDPE polymer in an amount of 0.1-15 % by weight of the fiber, in particular in an amount of 5-8 % by weight of the fiber.

Preferably, LDPE molecules have hexene or octene side chains, more preferably octene-side chains, are used as the anti-splicing agent, because this type of LDPE is particularly suited to increase entanglement thanks to the relatively long side chains.

According to embodiments, the artificial turf fiber comprises:
- 0.1-15%, in particular 5.0-8.0% by weight of low-density polyethylene (LDPE), and
- optionally 0.1-15% by weight HDPE; and
- optionally some further additives (pigments, lubricants) and/or fillers; and
- 60-99% by weight of linear low-density polyethylene (LLDPE). For example, the amount of LLDPE may be chosen such that the totality of LDPE and of the optional components such as HDPE, additives and fillers, if any, and he LLDPE add up to 100 % of the weight of the fiber (and, correspondingly, add up to 100% of the weight of the polymer mixture that is extruded into a monofilament to form the fiber.

According to embodiments, at least 60% by weight of the fiber, preferably at least 75% by weight of the fiber, comprises one or more polymers having a density in the range of 0.910 to 0.928 g/cm³, more preferably in the range of 0.913 to 0.925 g/cm³, and in particular in the range of 0.916-0.920 g/cm³.

For example, the fiber may comprise 0.1-15% LDPE having a density in a range of 0.919 g/cm³to 0.921 g/cm³, wherein the rest of the fiber material basically consists of an LLDPE having a density in a range of 0.918 g/cm3 to 0.920 g/cm³, e.g., 0.918 g/cm³.

For example, the artificial turf fiber may predominantly consist of LLDPE having a density in the above-specified range. The artificial turf fiber may also comprise LDPE acting as anti-splicing agent and preferably having a density in the specified density range.

This density range may provide additional protection from splicing: applicant has observed that fibers predominantly made of polymers with a lower density loose their helical form rapidly in response to wear and tear, and splice in longitudinal direction. On the other hand, polymers having a higher density tend to be more brittle and hence may have an increased risk of any kind of fiber breaks.

According to embodiments, the LLDPE polymer is a polymer created by a polymerization reaction under the presence of a Ziegler-Natta catalyst.

According to some embodiments, the Ziegler-Natta catalyst is a heterogeneous supported catalyst based on titanium compounds in combination with cocatalysts, e.g. organoaluminium compounds such as triethylaluminium.

According to other embodiments, the Ziegler-Natta catalyst is a homogeneous catalyst. Homogeneous catalysts are usually based on complexes of Ti, Zr or Hf and are preferentially used in combination with a different organoaluminium cocatalyst, methylaluminoxane (MAO). Using a Ziegler-Natta catalyst may have the advantage that the branches of the generated LLDPE are distributed more randomly, e.g. show an atactic orientation. This may ease the entanglement with branches of LDPE molecules.

According to embodiments, the LLDPE polymer is a polymer created by a polymerization reaction under the presence of a metallocene catalyst.

Using metallocene for catalyzing the polymerization for generating the LLDPE polymer may be advantageous as this particular form of catalysts ensures that the branching occurs in a less random and more defined manner. As a consequence of using metallocene as a catalyst, the number of branches per LLDPE molecule does not follow a normal distribution but rather follows a distribution having only one or very few (e.g. 1-3) peaks for the frequencies of branching per polymer molecule. Generating LLDPE polymers whose branch lengths are more randomly distributed may ease the entanglement with branches of LDPE molecules.

For example, a metallocene catalyst may be used together with a cocatalyst such as MAO, (AI(CH3)xOy)n. According to some examples, the metallocene catalyst has the composition Cp2MCl2 (M = Ti, Zr, Hf) such as titanocene dichloride. Typically, the organic ligands are derivatives of cyclopentadienyl. Depending on the type of their cyclopentadienyl ligands, for example by using an Ansa-bridge, metallocene catalysts can produce polymers of different tacticity and different branching frequencies. A tactic macromolecule in the IUPAC definition is a macromolecule in which essentially all the configurational (repeating) units are identical. The tacticity, branching frequency and distribution will have an effect on the physical properties of the polymer. The regularity of the macromolecular structure influences the degree to which it has rigid, crystalline long range order or flexible, amorphous long range disorder. According to embodiments, the tacticity of a polymer mixture that is used for manufacturing LLDPE or LDPE granules for use in artificial turf fiber production may be measured directly using proton or carbon-13 NMR. This technique enables quantification of the tacticity distribution by comparison of peak areas or integral ranges corresponding to known diads (r, m), triads (mm, rm+mr, rr) and/or higher order n-ads depending on spectral resolution. Other techniques that can be used for measuring tacticity include x-ray powder diffraction, secondary ion mass spectrometry (SIMS), vibrational spectroscopy (FTIR) and especially two-dimensional techniques.

According to embodiments, the LLDPE polymer is a polymer created by copolymerizing ethylene with 5 -12 % α-olefins having 3-8 carbon atoms, e.g. butene, hexene, or octene. The degree of crystallinity of the created LLDPE depends on the amount of added co-monomers and is typically in the range of only 30-40%, the crystalline melting range is typically in the range 121-125°C.

The production of LLDPE is initiated by a catalyst. The actual polymerization process can be done either in solution phase or in gas phase reactors. Usually, octene is the comonomer in solution phase while butene and hexene are copolymerized with ethylene in a gas phase reactor.

According to embodiments, the LLDPE polymer is a polymer comprising 0,001-10 tertiary C-atoms per 100 C atoms of the polymer chain. Preferably, the LLDPE polymer comprises 0.8- 5 tertiary C atoms /100 carbon atoms of the polymer chain.

According to embodiments, the LDPE polymer is a polymer more than 0.001, preferentially more than 1 tertiary C-atom/ 100 C atoms of the polymer chain.

The number of tertiary C-atoms is a measure of the degree of branching. Using an LLDPE and/or LDPE polymer having the above specified degree of branching may be advantageous as said degree of branching has been observed to cause a strong entanglement between LLDPE and LDPE polymer molecules which protects the polymer fiber against splicing. According to embodiments, manufacturing the artificial turf fiber comprises forming the stretched monofilament into a yarn. Multiple, for example 4 to 8 monofilaments, could be formed or finished into a yarn.

According to some embodiments, the artificial turf fiber has a cross-sectional profile with a central bulb and two wings extending in different, in particular opposite, directions.

For example, a cross-sectional fiber profile with a central bulb and two wings extending in different directions can be a shape with a thicker central core (the bulb) and thinner, elongated extensions (the wings) on either side. This type of fiber design has been observed to achieve a balance between strength, flexibility, and natural appearance and facilitates the introduction of stress which causes the material to acquire helical shape. Applicant has observed that other fiber profiles such as rhomboid or elliptic shapes are less suited for manufacturing helical fibers, may require different manufacturing parameters, and in some cases completely prevent the formation of helical structures.

The central bulb may act as the core of the fiber, giving it structural stability and strength. It is generally thicker than the wings, providing resistance to bending or flattening when the turf is subjected to foot traffic or other forms of stress. The bulb allows the fiber to remain upright, maintaining the artificial turf's appearance by preventing the fibers from easily bending or collapsing. The effect of the central bulb is further enhanced and stabilized by the helical structure.

The wings are thinner, flat extensions that branch out from the central bulb, usually in different, wherein the two arms typically form an angle in the range of 135°-225°, in particular 160° to 200°, e.g., 180°. These wings allow the fiber to move and flex when pressure is applied, giving the turf a more natural feel, similar to grass blades. The wings increase the surface area of each fiber, which can improve the light reflection properties, giving the turf a more natural look under different lighting conditions. The wings also contribute to the overall stability of the fiber by distributing pressure more evenly across the fiber, preventing localized wear and tear.

The combination of the central bulb and wings for a helical fiber provides excellent resistance to flattening, a common issue in artificial turf. This profile helps the fibers to acquire and maintain a helical 3D structure and to maintain an upright position even after prolonged use. This fiber profile is therefore particularly suited for use in high-performance artificial turf, especially for sports fields and high-traffic areas, due to its ability to withstand intense use while maintaining its shape and visual appeal.

According to embodiments, the artificial turf fiber profile has a core-cladding structure, wherein the core is embedded in the cladding. The fiber may comprise a central bulb which comprises the core. For example, a core-cladding structure can be formed using a co-extrusion nozzle configured to co-extrude a central polymer stream together with a cladding polymer stream that surrounds and contacts the core polymer stream during the extrusion process within the coextrusion nozzle. However, a fiber with a core polymer at the core and a different cladding polymer at the periphery can also be created with a single extrusion nozzle, e.g. via a three-phase system wherein the polymer beads comprising the core polymer will concentrate at the core because of the fluid dynamics during extrusion.

A core-cladding structure may be used, for example, for crating fibers in which the polymer material of the core is configured to shrink stronger upon receiving heat treatment than the polymer in the cladding. The heat-treatment may in particular be the heat treatment used for causing the extruded, quenched and stretched fiber to acquire the helical shape.

Applicant has observed that an artificial turf fiber profile with a core polymer that shrinks stronger than the cladding polymer upon heat treatment is adapted to strongly enhance the positive effect of the helical structure both in terms of mechanical strength and ability to re-erect after being trampled down. is the core polymer may be embedded within an outer layer formed by the cladding polymer. The central bulb of the fiber may serve as the primary load-bearing part of the fiber.

The core is the innermost part of the fiber. For example, the core may be defined by the crossing point of the longest fiber diameter and the shortest fiber diameter for a straight fiber cross-section. The core may be defined by the geometric center of a central bulb of a curved fiber cross-section.

The cladding polymer can be, for example, a polymer which is solely or predominantly (weight share of at least 60%) used in the periphery. The core polymer can be, for example, a polymer which is solely or predominantly (weight share of at least 60%) used in the core of the fiber cross section profile, in particular in the core of a central bulb. The periphery can be, for example, the regions in the fiber cross-section outside of the core. For example, the periphery may comprise the wings (if any), and may optionally also comprise a circular or elliptical region surrounding the core within a central bulb.

According to some embodiments, the core of a fiber is the central region within the cross-section of the fiber, while the periphery is the outermost region of the cross-section of the fiber. For example, the core may constitute about 10% to 70% of the cross-sectional area of the fiber, the rest of the cross-sectional area may belong to the periphery. In some embodiments, the core may constitute about 20% to 60% of the fiber, or 20% to 50%, depending on the geometry of the cross-sectional profile, e.g., the presence and length of the wings. The core may have various shapes, e.g., a circular, ellipsoid or other shape. The outer shape of the periphery may be defined by the outer shape of the cross-sectional profile of the fiber. In some embodiments, there polymer composition of the core and of the periphery may continuously merge into one other. For example, the core may comprise a higher concentration of a polymer material that shrinks stronger upon heat treatment than is comprised in the periphery, but both the core and the periphery may be made of the same base polymer. In other embodiments, the core may be made of a different polymer material than the periphery (e.g., as a result of co-extrusion of two different polymer masses). The periphery may form a ring or shell surrounding the core and extends to the external boundary of the cross-section.

According to embodiments, the core and periphery are two concentric regions within the cross-sectional fiber profile, with the core representing the inner area and the periphery representing the outermost area. There may not be a material boundary between these regions. If a material boundary is present, it may have various shapes depending for example on the dimensions of an inner path of a co-extrusion nozzle.

According to some embodiments, the core is made from a stronger, more rigid polymer that gives the fiber its mechanical strength and structural integrity. An example of such a polymer is polyamide. The purpose of the core provides rigidity and resistance to mechanical stress, helping the fiber stay upright and retain its shape under foot traffic or environmental pressure. The cladding polymer may be a softer or more flexible polymer compared to the core, which helps improve the flexibility and surface properties of the fiber. An example of this polymer is polyethylene, or a polyethylene comprising threads of a small amount of polyamide.

However, according to other embodiments which may have a particularly high splicing resistance and a particularly strong degree of twisting, the core polymer is a polymer or polymer mixture that exhibits greater shrinkage than the polymer material in the cladding in response to heat treatment. For example, the core polymer may be PE and the cladding polymer may be PA or PP. According to another embodiment which may also show improved splicing resistance and a strong degree of twisting, the core polymer may comprise a lower amount of HDPE than the cladding polymer, resulting in a greater shrinkage of the core polymer compared to the cladding polymer upon heat treatment. According to still another embodiment, the core polymer (before the fiber is extruded) may be a three-phase system comprising PE-beads embedded in a PA matrix, wherein the beads are surrounded by a compatibilizer, while the cladding polymer consists of PA or PP with no PE beads or a lower amount of PE beads than the core polymer.

According to some embodiments, the helical artificial turf fiber and the artificial turf made therefrom is used in high-traffic areas such as playgrounds and sports fields, such as soccer and rugby. The helical structure of the fiber, in combination with the anti-splicing agent and other optional features which may further reduce the risk of splicing may ensure that the artificial turf can withstand repeated use while maintaining its aesthetic appeal and functional performance for many years.

According to one example, the core is completely or predominantly made of an aged polymer, e.g., one or more aged polymers derived from aged and recycled artificial turf fibers, and the cladding is completely or predominantly made of a virgin polymer, e.g., a virgin polyamide and/or a virgin polyethylene.

According to some embodiments, the core polymer is a mixture of one or more aged polymers and a virgin polymer, wherein the virgin polymer is chosen such that the polymer mixture formed together with the aged polymer(s) is configured to shrink stronger in response to heat treatment than the virgin polymer in the cladding. For example, a first virgin polymer may be used for creating - together with the aged polymer - the core polymer, and a second virgin polymer may be used as the cladding polymer, whereby the first and the second virgin polymers are both polyethylene polymers, wherein the second virgin polymer has a higher amount of HDPE than the first virgin polymer (which may be free of the HDPE).

According to some embodiments, the artificial turf fiber comprises a hydrophilization agent.

A 'polymer material' as used herein is a material that consists of a single polymer, or a mixture of a polymer with one or more other substances. The other substances can be one or more additives and/or fillers and/or one or more other polymers. Hence, a polymer material may refer to a polymer or a polymer mixture.

A 'hydrophilization agent' as used herein is a chemical substance comprised in an artificial turf fiber (or in a polymer mixture used for manufacturing an artificial turf fiber) which is adapted to increase the affinity of the fiber for water. By modifying the surface properties of the fibers, hydrophilization agents make them more hydrophilic, meaning they are better able to adsorb or interact with moisture.

Many polymers used in the production of artificial turf fibers, such as polyethylene (PE), are inherently hydrophobic. As a result, when a hydrophobic fiber is removed from the water bath used for quenching, water forms droplets on the fiber surface and detaches quickly. This leads to rapid drying of the fiber surface.

To the contrary, an artificial turf fiber comprising an hydrophilization agent will adsorb more water and will keep the adsorbed water for a longer period of time when the fiber leaves the quenching water bath.

In the context of manufacturing helical fibers with a temperature gradient, keeping water attached to the surface for a longer duration of time may be highly advantageous, as it allows a more fine-granular control of the establishment of the temperature gradient: firstly, the water film on the surface of the fiber when it leaves the quenching bath may cool the fiber surface via evaporative cooling. This may help establish a radial temperature gradient. According to other embodiments, the water may be sprayed only to one side of the fiber, and the evaporative cooling may help establish the dorsoventral temperature gradient. The water film which may completely or partially cover the fiber surface may be combined with heating or cooling equipment (heated or cooled godets, heating or cooling elements along the transport path or in the stretching unit) and may increase the time span during which a temperature gradient can be established.

It should be noted that the cross-section of artificial turf fibers is very thin, so establishing a stable temperature gradient in a reproducible manner is a challenging task. Minor temperature differences in the environment, the quenching bath and/or in the equipment used for post-processing the extruded fiber, minor differences in the transportation speed, and other factors may have a strong impact on the stability of the amplitude and orientation of the temperature gradient. However, any distortions or partial breakdowns of the temperature gradient may result in the helix having incomplete or irregular rotation patterns. Hence, by adding a hydrophilization agent and ensuring that the extruded fiber is at least partially wetted with water while the temperature gradient is established may ease the control of the equipment such that a stable temperature gradient can be established reproducibly in the fiber cross section.

Examples of hydrophilization agents are surfactants, e.g., ionic and non-ionic surfactants such as polyethylene glycol (PEG). These surfactants reduce the surface tension of water, allowing the fibers to interact more effectively with moisture.

Another example for an hydrophilization agent is hydrophilic fumed silica, also known as hydrophilic pyrogenic silica. For example, fumed silica with the CAS number 112945-52-5 may be used. Fumed silica consists of microscopic droplets of amorphous silica fused into branched, chainlike, three-dimensional secondary particles which then agglomerate into tertiary particles. The resulting powder has an extremely low bulk density and high surface area. Its three-dimensional structure results in viscosity-increasing, thixotropic behavior when used as a thickener or reinforcing filler, and it also increases the hydrophily of the artificial turf fiber.

According to some embodiments, the artificial turf fiber comprises a nucleating agent.

A nucleating agent as used herein refers to a substance added to the polymer material during the production process to promote the formation of crystalline regions in the polymer matrix. These crystalline regions, or nuclei, serve as the initial points from which further crystallization can grow during cooling or solidification of the polymer. Nucleating agents are particularly useful in increasing the surface roughness of artificial turf fibers.

The nucleating agent can be an inorganic and/or an organic substance or a mixture thereof. For example, the inorganic substance acting as the nucleating agent can consists of one or more of the following: talcum; kaolin; calcium carbonate; magnesium carbonate; silicate; silicic acid; silicic acid ester; aluminum trihydrate; magnesium hydroxide; meta- and/or polyphosphates; and coal fly ash. Fumed silica is a further inorganic nucleating agent.

Examples of organic substances acting as the nucleating agent may be one or more of the following: 1,2-cyclohexane dicarbonic acid salt; benzoic acid; benzoic acid salt; sorbic acid; and sorbic acid salt.

The nucleating agent may have the advantage of increasing the surface roughness, thereby increasing the surface of the fiber, and hence, increasing the amount of water that can adhere to the surface of the fiber (per fiber length unit).

The use of hydrophilic fumed silica is particularly advantageous in this context, as it functions both as a hydrophilizing agent and a nucleating agent. This dual functionality makes it especially effective in increasing the amount of water that adheres to the fiber surface, thereby greatly facilitating the control and maintenance of temperature gradient formation.

In a further aspect, the invention relates to an artificial turf. The artificial turf comprises a carrier and a plurality of artificial turf fibers. The fibers are integrated into the carrier and extend to one side of the carrier. The artificial turf fibers comprise an anti-splicing agent and comprises stress which, upon receiving heat treatment, causes the artificial turf fiber to change its 3D shape to form a helical artificial turf fiber. For example, the artificial turf may be greige good in the state before it is head-treated to cause the artificial turf fiber to change its 3D shape to form a helical artificial turf fiber. In the context of artificial turf, the term "greige good" refers to the unfinished synthetic fabric that already comprises a carrier with integrated fibers. It has not yet undergone additional processes like coating, final heat-treatment or adding infill, which are necessary to complete the artificial turf product.

In a further aspect, the invention relates to an artificial turf. The artificial turf comprises a carrier and a plurality of helical artificial turf fibers according to any one of the embodiments described therein. The fibers are integrated into the carrier and extend to one side of the carrier.

For example, the artificial turf may be an artificial turf ready for installation or an already installed artificial turf.

According to embodiments, the artificial turf is an infill-less artificial turf. Thanks to the helical structure, which provides a particularly high elasticity and resilience, adding infill may often not be necessary, thereby increasing the recyclability of the artificial turf.

According to other embodiments, the artificial turf comprises infill. The infill can be, for example, sand, rubber particles, organic particles such as cork or olive pit particles, or mixtures of two or more of these infill types.

According to embodiments, the helical artificial turf fibers are non-texturized fibers. This may have the advantage of providing a layer of helical fibers having a more clearly defined, and a more homogeneously distributed, layer-height measured from the carrier layer to the tips of the helical fibers.

According to embodiments, the helical artificial turf fibers are texturized fibers.

According to some examples, the artificial turf comprises helical, texturized fibers and non-texturized fibers. In particular, the non-texturized fibers may approximately have the same length or a larger length than the helical, texturized fibers.

For instance, the helical fibers can be utilized to stabilize and secure the infill, with their length slightly exceeding the height of the infill layer. In contrast, the non-helical fibers may be longer and serve to enhance the natural appearance and texture of the turf.

A 'texturized fiber' as used herein refers to synthetic grass fibers that have been processed to create a crimped or curled texture. This texture may increase durability, reduces glare, and helps the fibers stand upright, giving the turf a more natural look and feel while also improving its performance in applications like sports fields or landscaping. The texturizing process has been observed by the applicant to enhance the turf's ability to resist wear and maintain consistent appearance over time.

According to embodiments, the artificial further comprises an infill layer. The number of twists per fiber and the height of the infill is chosen such that in at least 80% of the helical fibers, the part of the helical fibers that protrudes above the infill layer makes at least 1.0 turns.

This may have the benefit of preventing splashing of the infill particles.

According to one example, the straight, non-texturized and non-helical fibers have a height in the range of 2.5 cm - 8.0 cm, preferably 4.0 cm - 6.0 cm. The texturized and helical fibers have a height (in helical shape, i.e., after heat-treatment) in the range of 1.5 cm - 8.0 cm, preferably 1.5-6.0 cm. According to some embodiments, the length of the helical fiber is about 80% to 90% the length of the non-texturized, non-helical fiber. The infill layer has a height in the range of 80% to 90% the length of the helical fiber. The length or height are measured from the upper surface of the carrier into which the fibers are integrated.

In a further aspect, the invention relates to a method for manufacturing an artificial turf fiber which comprises stress which, upon receiving heat treatment, causing the artificial turf fiber change its 3D shape to form a helical artificial turf fiber. The method comprises:
- creating a polymer mixture, the mixture comprising an anti-splicing agent:
- extruding the polymer mixture into a monofilament;
- quenching the monofilament;
- controlling equipment for processing the monofilament such that a temperature gradient is formed reproducibly in the cross-section of the monofilament;
- stretching the monofilament while the temperature gradient is present to form the monofilament into the artificial turf fiber, thereby introducing the stress into the fiber material.

In a further aspect, the invention relates to a method for manufacturing a helical artificial turf fiber. The method comprises:
- creating a polymer mixture, the mixture comprising an anti-splicing agent:
- extruding the polymer mixture into a monofilament;
- quenching the monofilament;
- controlling equipment for processing the monofilament such that a temperature gradient is formed reproducibly in the cross-section of the monofilament;
- stretching the monofilament while the temperature gradient is present to form the monofilament into the artificial turf fiber, thereby introducing the stress into the fiber material; and
- heating the monofilament, thereby causing the artificial turf fiber change its 3D shape to form a helical artificial turf fiber.

According to embodiments, the extruding is performed via an extrusion channel having a capillary length of less than 2.5 mm, in particular a capillary length of less than 2.0 mm, in particular of 1.4 to 1.6 mm. Using a short capillary length may have the advantage of promoting turbulent flow during the extrusion process, thereby preventing a predominantly lateral orientation of the polymer molecules, which could increase the splicing risk.

According to embodiments, the stretching is performed such that the ratio of the fiber length before stretching to the fiber length after stretching falls within the range of 1:4.0 to 1:5.1. The applicant has observed that this stretching range offers an optimal balance between brittleness and the number of helical turns that can be introduced into the fiber. If the degree of stretching is too high, the fiber may become brittle due to an excessive formation of crystalline regions. Conversely, if the stretching is insufficient, the fiber may not develop a pronounced helical 3D shape in response to heat treatment.

According to embodiments, the temperature gradient is a dorso-ventral gradient or a radial gradient.

According to embodiments, the fiber has a cross-sectional profile with a maximum-length-to-maximum-thickness ratio of at least 3.0, or of at least 4.0, or of at least 5.0, and wherein in particular the temperature gradient is a radial gradient.

An 'anti-splicing agent' as used herein is an agent, i.e., a substance or a combination of substances, that reduces the likelihood of fiber splicing. Splicing refers to the creation of cracks in longitudinal direction of a fiber, which have been observed to occur particularly soon in helical fibers. Without the intention to be bound by any theory, applicant assumes that these cracks are caused by the stress having been introduced into the material for causing the fiber to acquire helical shape.

A 'helical' fiber as used herein is a helical fiber in the strict sense, and/or is a twisted fiber. A twisted fiber is a fiber rotated around its longitudinal central axis. While a helix in the strict, mathematical sense rotates around the central axis of a cylinder, a helical fiber as used herein is to be construed broadly to cover both helical fibers in the strictly mathematical sense, and twisted fibers having only a rotational motion along is own central longitudinal axis without any translational movement. The term also covers fibers which are both helical in the strict sense and in addition are twisted around their central longitudinal axis.

The stress which, upon receiving heat treatment, may cause the fiber to acquire helical structure may be, for example, tensile and/or compressive stress that develops during the manufacturing process, such as extrusion, stretching, or cooling. This stress arises from uneven distribution of molecular alignment or crystallization within the fiber. As the fiber relaxes, e.g., upon heat treatment, these built-up stresses are released, causing the fiber to twist, coil, or take on a helical shape. By controlling manufacturing equipment such that a temperature gradient is generated across the fiber profile in a controlled and reproducible manner (both in terms of the amplitude and the orientation of the gradient) and such that the fiber is compressed and/or stretched in a controlled, reproducible manner while the temperature gradient is present, stress can be introduced in a controlled and reproducible manner such that helical fibers with a defined number of turns are formed later in response to a heat treatment.

### BRIEF DESCRIPTION OF THE DRAWINGS

In the following, examples are described in greater detail making reference to the drawings in which:
Fig. 1 is a flow chart of a method of manufacturing helical artificial turf fibers;
Fig. 2 is a diagram illustrating artificial turf comprising a helical, texturized and a straight fiber;
Fig. 3 is a photo of an artificial turf comprising a helical, texturized and a straight fiber;
Fig. 4 is a photo of an artificial turf comprising a helical, texturized and a straight fiber and infill;
Fig. 5 is a collection of cross-sectional profiles of a helical fiber;
Fig. 6 is a photo of a Lisport Test apparatus adapted to test mechanical fiber stability in many thousand test cycles;
Fig. 7 is a photo of two different types of artificial turf;
Fig. 8 shows photos of the two different types of artificial turf and a helical artificial turf fiber comprised therein after 0 cycles;
Fig. 9 shows photos of the two different types of helical artificial turf fiber after different numbers of cycles;
Fig. 10 is a photo of the artificial turf shown in figure 4 after 50.000 cycles; and
Fig. 11 is an illustration of the impact of the LLDPE anti-splicing agent on the extrusion process;
Fig. 12 is a cross sectional drawing of a co-extrusion device used for the manufacturing of artificial turf fibers with a core-cladding structure;
Fig. 13 shows different types of temperature gradients used for inducing a helical structures in different groups of fiber profiles;
Fig. 14 is a block diagram of a manufacturing line for manufacturing artificial turf yarn;
Fig. 15 is a photo of a cross-section of a fiber; and
Fig. 16 shows various cross-sectional fiber profiles.

### DETAILED DESCRIPTION

In the following, similar elements are denoted by the same reference numerals.

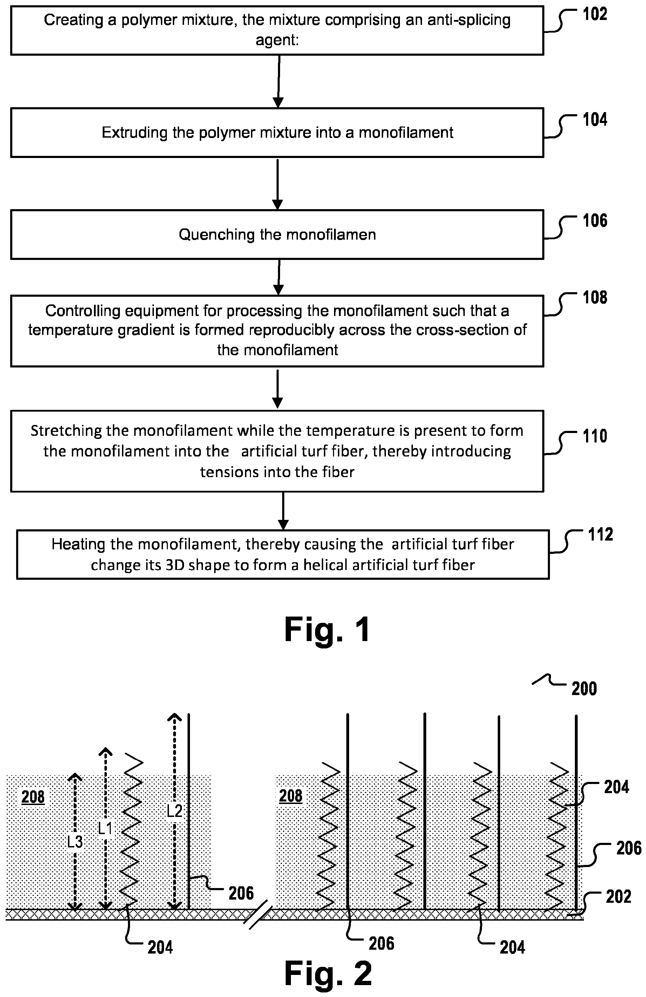

**Figure 1** shows a flow chart of a method of manufacturing artificial turf fibers comprising stress which, upon receiving heat treatment, causes the artificial turf fiber change its 3D shape to form a helical artificial turf fiber (steps 102 to 110), and for manufacturing helical artificial turf fibers (step 112). The steps in this process are as follows:

In a first step 102, a polymer mixture is created. The polymer mixture is formulated with an anti-splicing agent. The mixture serves as the raw material for the artificial turf fibers, with the anti-splicing agent playing a crucial role in preventing the fiber from splitting or fraying under mechanical stress.

A base polymer is selected that will form the bulk of the artificial turf fiber. Preferably, polyethylene (PE) is chosen for its softness, flexibility, and UV resistance. In some embodiments, the PE may be or comprise low-density polyethylene (LDPE) or high-density polyethylene (HDPE). In applications requiring greater durability or heat resistance, polypropylene (PP) or polyamide (PA) may be used as base polymer. The polymer(s) are mixed with the anti-Splicing agent(s) to prevent the fiber from splitting or unraveling under mechanical stress. The anti-splicing agent may comprise, for example, elastomers or other substances that provide flexibility and bond strength within the fiber matrix. In particular, a compatibilizer and/or a significant portion of LLDPE may be used as anti-splicing agent.

Various further additives may be incorporated into the mixture before extrusion: UV Stabilizers, Colorants, processing aids (like lubricants and stabilizers that help improve the flow properties of the polymer during extrusion, reducing friction and preventing thermal degradation) and/or anti-static agents may be used.

Once the base polymer and additives are selected, the materials are combined in a melt mixing process. This typically occurs in an industrial extruder where the polymer granules and additives are fed into the extruder's barrel and mixed together under heat and pressure. The extruder temperature is precisely controlled to ensure proper melting and blending of the polymer and additives. Typical processing temperatures range from: Polyethylene (LDPE/HDPE): 160-250°C, Polypropylene: 180-280°C and Polyamide (Nylon): 230-290°C. The mixing speed and time is chosen as to ensure uniform blending of polymers and additives.

In a next step 104, the polymer mixture is extruded to form a monofilament. In this step, the polymer is pushed through a die, resulting in a continuous, uniform fiber that will be further processed. The extrusion defines the initial dimensions (cross sectional fiber profile) and properties of the fiber.

After extrusion, the monofilament undergoes rapid cooling, also referred to as quenching 106. This step solidifies the fiber and helps lock in its shape, establishing its basic structural properties. Quenching is preferably performed in a water bath set to a particular temperature, e.g., about 4°-20°. The temperature of the water bath and/or the temperature of godets used for transporting the fibers from the water bath to other fiber processing units may be set to a defined temperature chosen such as to ensure the formation of a stable, reproducible temperature gradient. The temperature differential is key to inducing internal stresses that lead to the fiber's twisting or coiling behavior. The temperature gradient ensures that the stretching will introduce stress in the fiber, which will finally result in the formation of a helical structure in response to heat treatment.

Next in step 108, equipment used for processing the extruded fiber is controlled such that a temperature gradient is formed reproducible across the cross-section of the extruded monofilament. For example, the temperature of the quenching bath, the temperature of godets used for transporting the fiber from the quenching unit to a stretching unit, to a texturization unit and/or to a winding unit for winding the fiber onto a bobbin, are chosen such that the temperature gradient is formed during transportation and/or while the fiber is processed in the stretching chamber.

Next in step 110, the monofilament is stretched while the temperature gradient is present. Stretching the fiber will increase its length and enhance the proportion of crystalline regions relative to amorphous regions in the polymer material. This stretching process also introduces stress into the fiber.

Introducing stress during the transport and/or during the stretching while the temperature gradient is present will usually not immediately result in a helical structure, but will cause the fiber to adapt helical shape upon heat treatment. The orientation of the temperature gradient may be chosen in dependence of the cross-sectional profile of the fiber. For example, a dorsoventral gradient may be chosen for fibers with a maximum-length to maximum-thickness ratio in the cross-sectional profile of below 3.0, while a central-radial temperature gradient may be introduced for fibers whose ratio is 3.0 or higher.

Preferably, the equipment used for fiber processing is controlled such that both the height and orientation of the temperature gradient and the amplitude and direction of the mechanical stress applied while the temperature gradient is present are applied in a defined, controlled and reproducible manner as to ensure a regular periodicity of the turns of the helix to be created in response to heat-treatment.

For example, the stretching may be performed by using godets with differential speed such that the stretching is executed during the transport of the fiber. In addition, or alternatively, the stretching may be performed in a stretching chamber. This stretching process not only strengthens the fiber but also primes it for taking on its helical shape.

After stretching, the monofilament is heated in step 112. The heating step may comprise heating the artificial turf fiber (or the artificial turf or greige good comprising the same) to a temperature above 70°C, in particular a temperature between 70 °C and 130°C, in particular a temperature between 80°C and 90°C. The heating step will cause the fiber to change it's 3D shape into a helical configuration.

**Figure 2** is a detailed cross-sectional representation of an artificial turf 200 comprising a helical, texturized fiber 204 and a straight fiber 208. The artificial turf also comprises an infill layer 208. This configuration is designed to enhance the durability, aesthetic appeal, and functionality of the artificial turf.

A carrier 202 is a sheet-like structure for carrying the artificial turf fibers. The carrier layer serves as the foundation into which the fibers (both helical and non-helical) are anchored, providing structural stability to the turf system. The carrier is typically composed of a flexible yet durable material that supports the fibers and accommodates the infill layer. For example, the carrier can be a mesh, e.g., a methos of synthetic and/or natural fibers.

Helical, texturized fibers 204 are shown extending upward from the carrier 202. These fibers are formed in a twisted, helical shape to mimic the appearance of natural grass blades and offer enhanced resilience. The helical shape provides elasticity and helps maintain the turf's upright position, ensuring that the turf retains its appearance and cushioning properties even after repeated use. The helical shape also allows to keep infill particles in place.

The length of the helical fibers 204 is indicated as L1, representing the vertical extension from the carrier to the top of the helical fiber. These fibers typically extend to a height that contributes to the overall softness and bounce of the turf and that is preferably higher than the height L3 of the infill layer 208.

Alongside the helical fibers, the turf 200 also comprises non-helical, straight fibers 206. These fibers contribute to the fiber's optical properties. The length of the non-helical fibers is denoted as L2, which may vary depending on the design of the turf. These straight fibers in the depicted example extend higher as the helical fibers, contributing to a balanced appearance and feel.

Surrounding and partially covering both the helical and non-helical fibers is the infill layer 208. The infill is typically composed of granular materials such as rubber or sand, which help to provide cushioning, stability, and weight to the turf system. The infill also contributes to the overall performance of the turf, aiding in shock absorption and maintaining the vertical alignment of the fibers. The infill layer extends to a height indicated as L3, filling the space between and around the fibers. The height of the infill layer is carefully controlled to ensure it provides adequate support while allowing the fibers to extend above it for a natural look and feel. In particular, the number of turns in the helical fibers, the height of the infill L3 and the height L1 of the helical fibers are chosen such that the helical fibers extend at least 1 whole turn, preferably at least 1.5 to two turns above the infill. This is to ensure that the helical fibers can sufficiently contribute to optical and playing properties of the artificial turf such that the turf does not have grain.

A common problem of artificial turf is that the synthetic fibers forming the artificial turf surface have a grain. The grain of an artificial turf as used herein encompasses the natural tendency of turf fibers to lie at a slightly slanted angle. The term grain is also alternatively known as the pile direction. In German the grain is known as Florrichtung. The "grain" of artificial turf refers to the direction in which the synthetic grass blades are oriented and lie. The grain of artificial turf affects its appearance, texture, and performance, and is typically undesired: the grain may have negative effects on the visual appearance, as the turf can look patchy or show stripes due to differences in how light reflects off the fibers when viewed from different angles. In can also contribute to inhomogeneous ball roll characteristics and even an increased risk of injuries for the players. That L1, L3 and the manufacturing properties influencing the number of terns per length unit of the helical fibers are chosen as specified above ensures that the twisted, helical nature of the helical fibers can have a sufficient effect on the optical properties and playing characteristics.

The helical fibers may be integrated into other types of artificial turf as well. For example, they may be integrated into an infill-less artificial turf. They may be helical and texturized or helical and non-texturized. The helical fibers may be the only type of fibers in the turf, or they may be combined with other fiber types, e.g. non-helical texturized or non-helical non-texturized fibers.

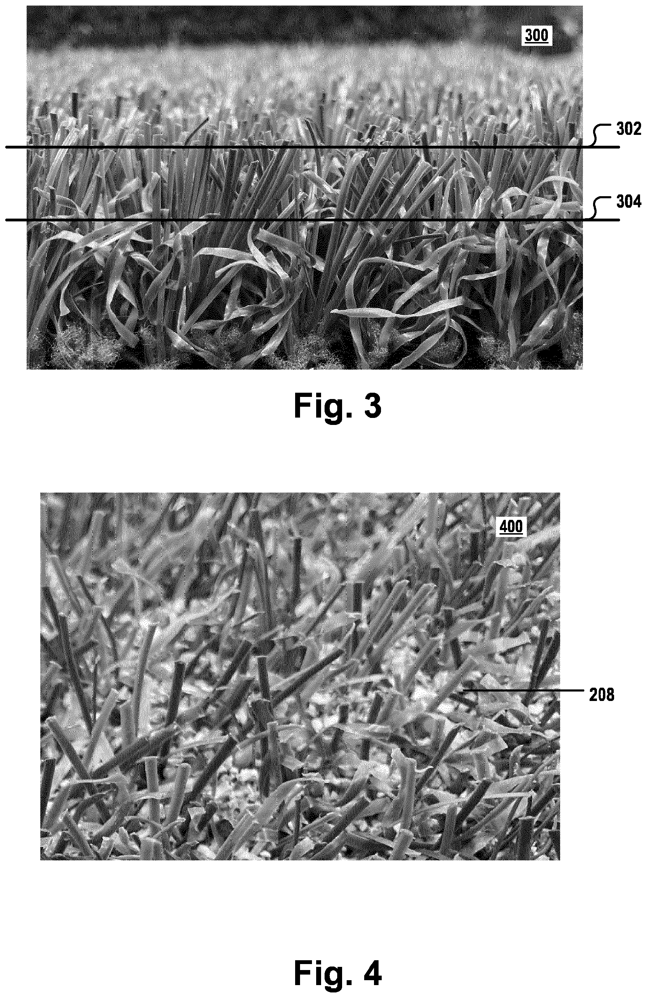

Figure 3 is a photo of an artificial turf 300 comprising a helical, texturized and a straight fiber. The line 302 indicates the height L2 of the non-helical, straight fibers, and the line 304 indicates the length L1 of the texturized and helical fibers.

**Figure 4** is a photo of an artificial turf 400 comprising a helical, texturized fiber and a straight fiber and infill. For example, the turf 400 may be created by adding infill to turf 300 after installation at the use site.

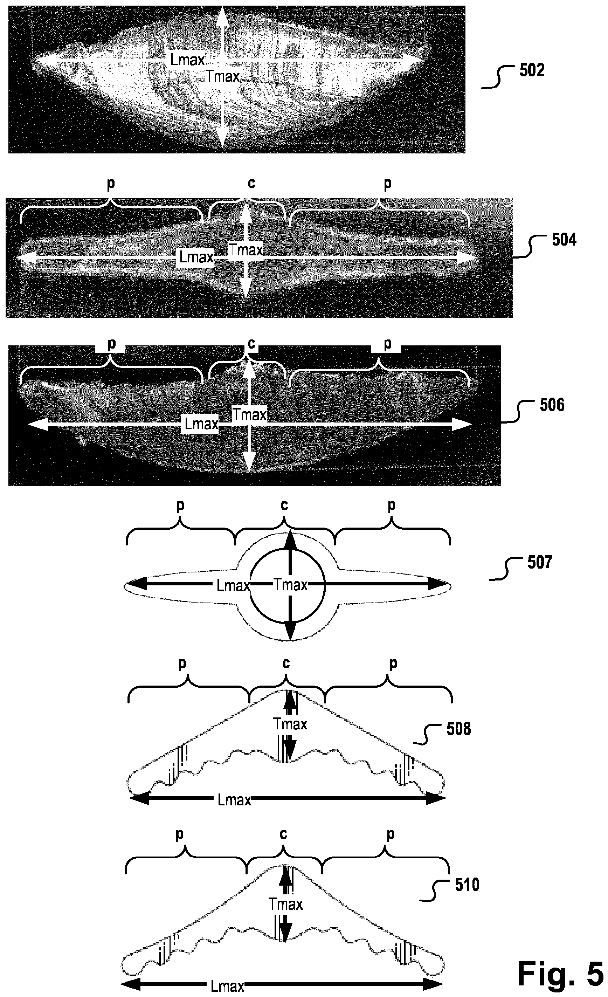

**Figure 5** is a collection of cross-sectional profiles 502-510 of different helical fibers. Applicant has observed that the cross-sectional fiber profile has a strong impact on the capability of a fiber to acquire a helical shape, and has developed different, profile-specific techniques for integrating stress into the fiber profiles in order to force the fibers to acquire helical shape. The different types of profiles, their impact on the ability to form helical structures, and suggested procedural adaptations of the manufacturing process for generating artificial turf fibers having a helical structure will be discussed in the following.

Diamond/Rhomboid profiles: Fiber profile 502 is an example of a fiber profile with longitudinal shape having a central bulb with wings (protrusions) extending in different directions, wherein the fFiber profile 502 has a maximum length (Lmax) to maximum thickness (Tmax) ratio of approximately 2.5.

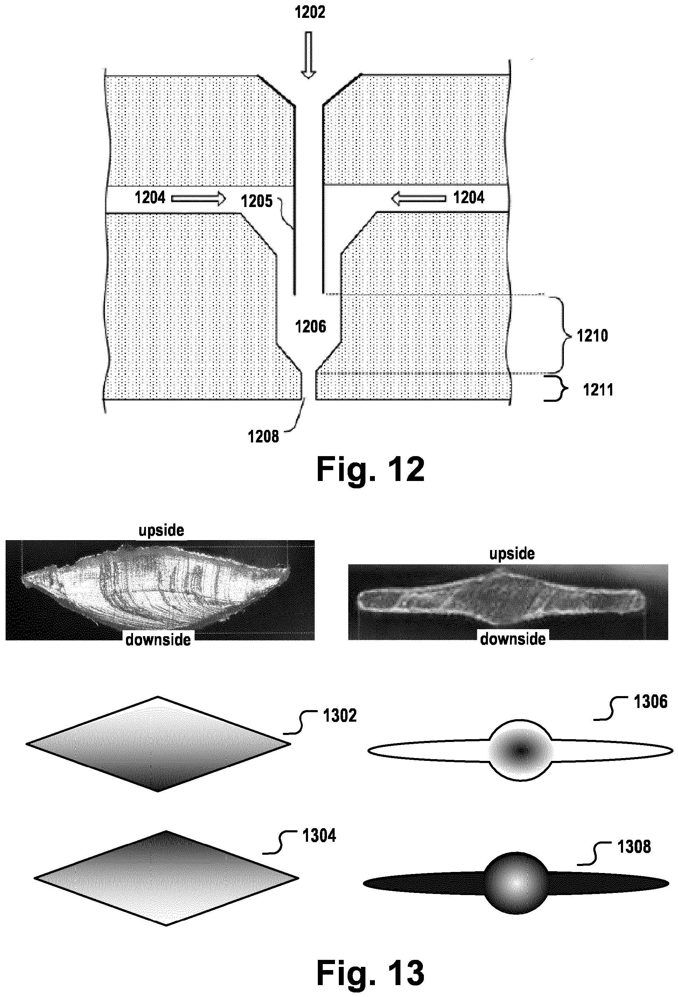

The applicant has observed that for fiber profiles with a maximum length to maximum thickness ratio of less than 3.0, a "dorsoventral" temperature gradient (1302, 1304) across the fiber's cross section, as shown in Figure 13, during stretching or compression is preferred. This temperature gradient may ensure that the fibers will adopt a helical structure when subjected to heat treatment. A radial temperature gradient may not be sufficient to induce a strong helical structure as any induced length difference between the core and the peripheral regions may not be sufficient to overcome the rigidity imposed by the massive central region. Hence, a dorso-ventral temperature gradient is preferred for this type of fiber profile.

Fiber profiles 504-510 are examples of longitudinal artificial turf fiber profiles that feature a central bulb with wings extending in different directions which have a cross-sectional profile with a maximum length to maximum thickness ratio greater than 3.0. In certain embodiments, fiber profiles with a central bulb and wings extending therefrom in different directions, particularly those with a maximum length to maximum thickness ratio of 3.0 or higher, are induced to form helical structures by establishing a core-to-radial temperature gradient (1306, 1308), as illustrated in Figure 13. In all fiber profiles 504-510, the maximum length is indicated with the label Lmax, the maximum thickness (or width) is indicated with the label Tmax, the central bulb is indicated with the label "c" and the wings (protrusions) extending therefrom are indicated with the label "p".

The profiles 504- 510 are particularly suitable for manufacturing helical fibers as these profiles have been observed to facilitate and boost the formation of a helical structure, possibly because the wings are comparatively thin compared to the thickness of the central bulb, so the stress having been introduced into the polymer material faces less resistance by the polymer mass of these whole fiber cross section.

Fiber profiles 504, 506-510 may be manufactured using a single extrusion nozzle or using a co-extrusion nozzle as illustrated in figure 12. If a co-extrusion nozzle is used, the fiber may have a core-cladding structure. The core-cladding structure may in some cases be easily visible if different dyes are used for the cladding and the core polymer, and may in other cases be determinable by a more detailed optical or chemical analysis of the polymer material. The fiber profile 507 is an example of a fiber having a core-cladding structure as a result of a co-extrusion process, wherein the core polymer is visibly separated from the cladding polymer.

Fiber profile 507 comprises a cylindrical core and a non-circular cladding surrounding the core. The core comprises a core polymer. In some embodiments, the core polymer is a blend of an aged polymer and a virgin polymer. In addition, or alternatively, the core polymer may be a polymer material that will shrink stronger than the cladding polymer upon heat treatment. The heat treatment can be, for example, a step of heating the artificial turf after having applied a liquid polyurethane or latex mass or other type of liquid backing material on order to harden and solidify the liquid backing.

The core polymer may be a polymer which is miscible with the cladding polymer. For example, the core polymer can be polyethylene and the cladding polymer can be polyamide or polypropylene. The use of a compatibilizer as an anti-splicing agent may be particularly useful for core-cladding fiber profiles having different, immiscible core and cladding polymers, as the compatibilizer helps to prevent delamination and splicing at the contact surface of the core and cladding polymer.

The core may for instance have a diameter of 50 to 600 micrometer in size. It may typically reach a yarn weight of 50 to 3000 dtex.

In other embodiments, the cladding is formed by a cladding polymer which is chosen to be miscible with the core polymer in fluid state. The cladding polymer may be identical to the core polymer. The annular cylindrical zone or area where the cladding polymer contacts the core polymer is a contact layer where both polymers are mixed with each other. Hence, the contact layer may bond core and cladding together with stronger forces than the long-range forces which occur typically within arrangements with a purely cohesive bonding.

The cladding completely surrounds the core with two circular sections on two opposite sides of the core and two flat, thin, long wings on two other opposing sides of the core.

The cladding may be based on a polymer such as polyethylene which may provide a soft and smooth surface characteristic, or on polyamide which has the benefit of a reduced shrinkage in response to heat treatment relative to a PE-based core polymer. The cladding may comprise additives which support its interfacing function to the environment and / or a user. These additives to the cladding may be, for example, pigments providing a specific color, a dulling agent, a UV stabilizer, flame retardant materials such as aramid fibers or intumescent additives, an anti-oxidant, a fungicide, and / or waxes increasing the softness of the cladding.

Providing the cladding with additives may have the advantage that these can be left out from the core. This way, a smaller content of expensive additive material per mass unit is required. As an example, it is not necessary to add pigments to the core because only the cladding is visible from the outside. By way of a more specific example, it may be beneficial to add a green pigment, a dying agent and a wax to the cladding to gain a closer resemblance of natural grass blades.

Preferably, the cladding resembles a blade of grass by encompassing the circular-cylindrical core with two wings extending in two opposite directions from the geometric center of the monofilament.

A fiber 507 having a core-cladding structure can be produced by feeding a core polymer mixture and a cladding polymer component into a fiber producing coextrusion line. The two polymer melt components are prepared separate from each other and then joined together in the coextrusion tool, i.e., a spinneret plate, forming the two melt flows into a filament which is quenched or cooled in a water spin bath, dried and stretched by passing rotating heated godets with different rotational speed and / or a heating oven.

The two wings may have various forms and orientations. For example, the wings in profile 504 and 508 protrude in opposite (180°) directions while the wings in profiles 508 and 510 protrude in an angle of about 135 °. The wings of profile 508 have an undulated and a non-undulated, straight side, while the wings of profile 510 have an undulated side and a non-undulated, concave side.

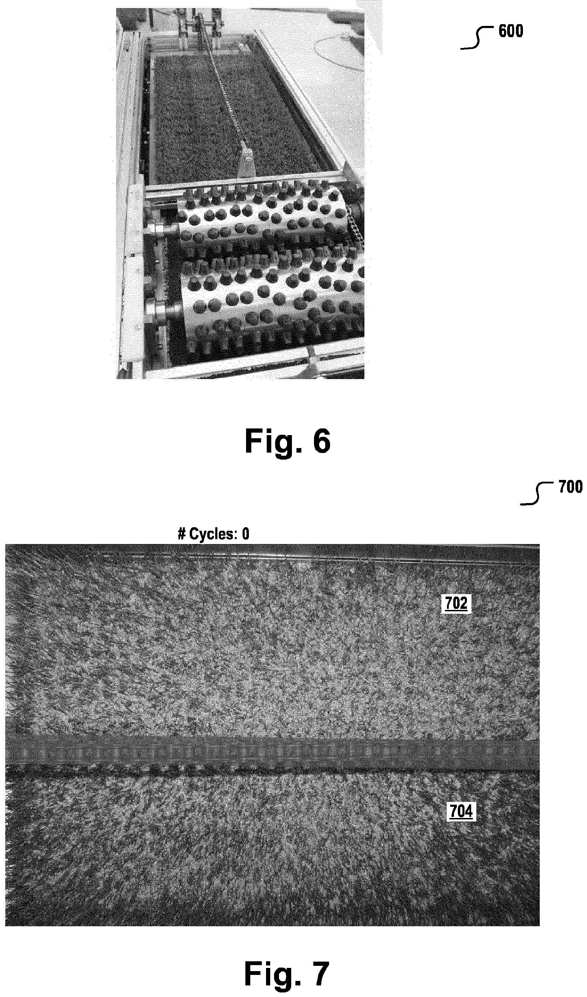

**Figure 6** is a photo of a Lisport Test apparatus adapted to test mechanical fiber stability in many thousand test cycles. The Lisport Test is a specialized testing method used to assess the durability and wear resistance of artificial turf. The test simulates the effects of long-term use on artificial turf by subjecting it to repeated mechanical abrasion and impact, mimicking the conditions experienced in real-life scenarios over several years. A machine equipped with rollers or studs repeatedly moves across the surface of the artificial turf, exerting pressure and friction, similar to the effect of people walking, running, or playing on the turf. The test is conducted in cycles, and the number of cycles simulates the number of years of use. A standard Lisport Test might simulate several years of wear in a matter of hours or days. After the test, the turf is evaluated for wear, such as fiber flattening, splitting, or loss of resilience. This helps manufacturers and users understand how the turf will hold up under long-term usage. The Lisport Test is an industry-standard test for evaluating artificial turf's durability (FIFA QUALITY PROGRAMME FOR FOOTBALL TURF, TEST MANUAL I: TEST METHODS, April 2024 edition, p. 52).

**Figure 7** is a photo of an area 700 that shall undergo a Lisport test. The upper half of the area is covered with artificial turf incorporating helical fibers that include an anti-splicing agent (in the following anti-splicing(+) turf), while the lower half is covered with artificial turf containing helical fibers manufactured without the anti-splicing agent (in the following anti-splicing(-) turf).

The compositions of the two compared turfs are as follows:

| | **Anti-splicing(+) turf** | **Anti-splicing(-) turf** |
|---|---|---|
| **Polymer** | 83 % by weight LLDPE, density: 0.918 g/cm³ | 95% by weight LLDPE, density: 0.918 g/cm³ |
| **Anti-splicing agent 1** | 2% by weight: Compatibilizer (Maleic Acid Anhydride grafted PE) | - |
| **Anti-splicing agent 2** | 10 % by weight LDPE | - |
| **Further additives (UV-stabilizer, pigments)** | 5 % by weight of the fiber | 5 % by weight of the fiber |

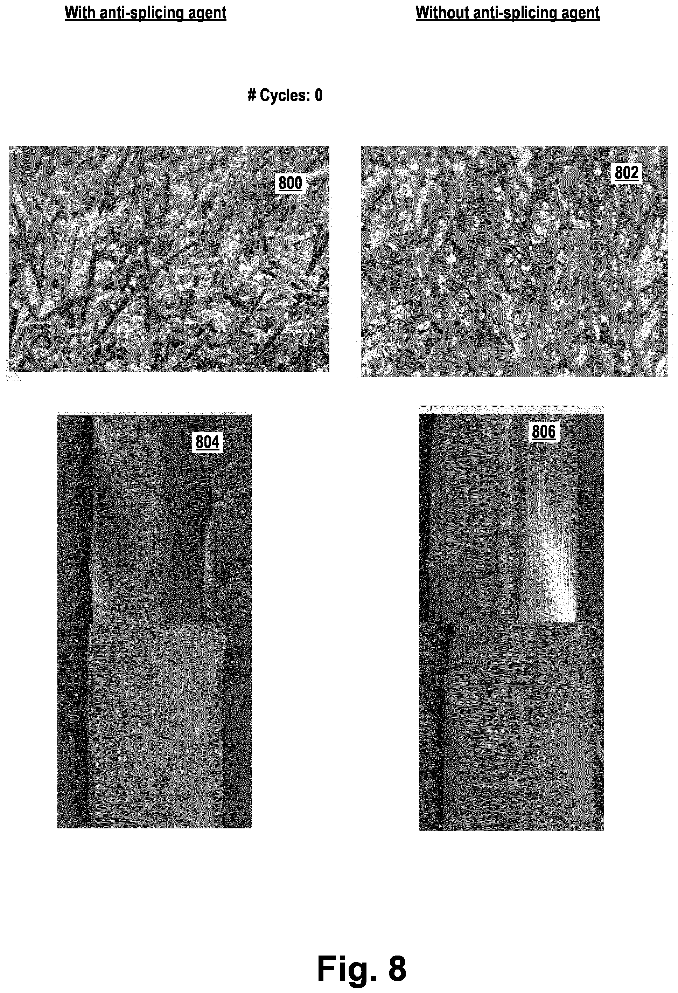

**Figure 8** shows a photo 800 of artificial turf comprising helical anti-splicing(+) turf fibers one of which being depicted in greater detail in photo 804 after 0 cycles of the Lisport test. Figure 8 also shows a photo 802 of artificial turf comprising helical anti-splicing(-) turf fibers one of which being depicted in greater detail in photo 806 after 0 cycles of the Lisport test. As can be seen, both types of fibers look intact and do not show any signs of splicing.

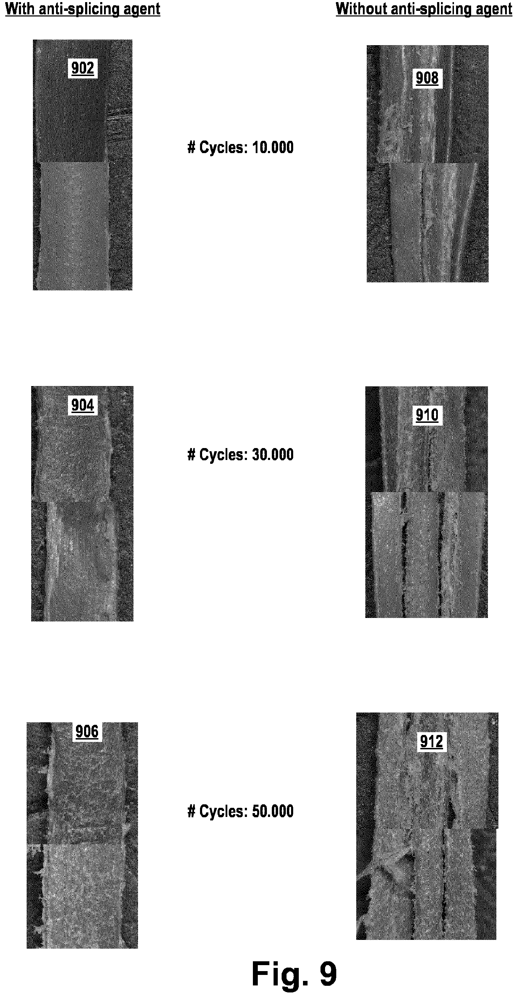

**Figure 9** shows photos 902-906 of the helical anti-splicing(+) turf fibers after 10.000, 30.00 and 50.000 Lisport test cycles and photos 908-912 of the helical anti-splicing(-) turf fibers after 10.000, 30.00 and 50.000 Lisport test cycles. As can be seen from the photos, the helical fiber without the anti-splicing agent is severely damaged by lateral splicing already after 10.000 cycles, and is completely destroyed after 50.000 cycles, while the artificial turf fiber comprising the anti-splicing agent is still intact and does not show signs of splicing.

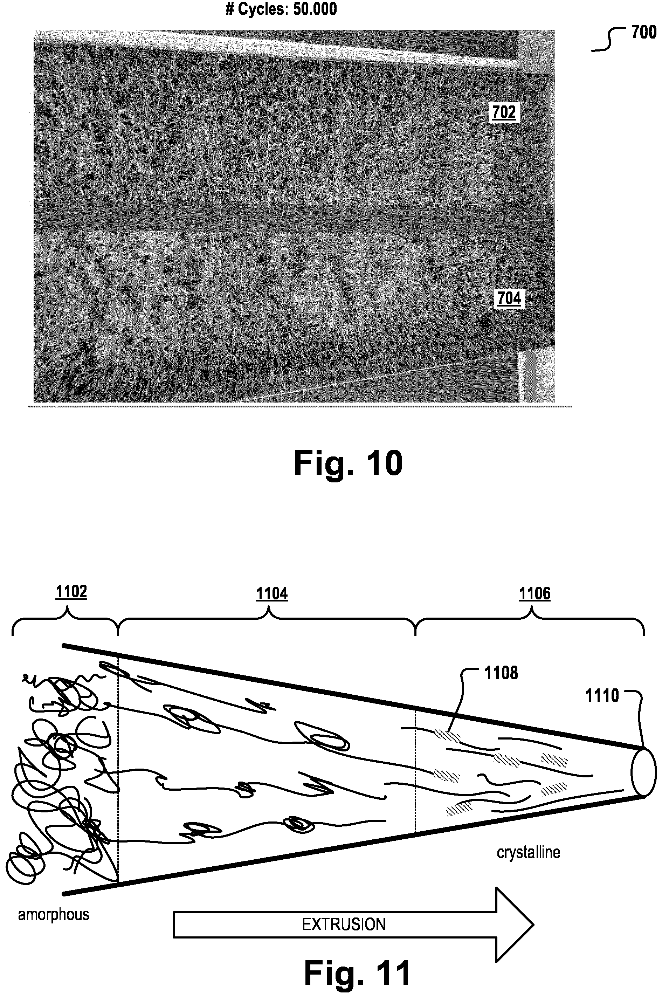

**Figure 10** is a photo of the artificial turf 700 shown in figure 7 after 50.000 cycles. It can be seen that the artificial turf 702 comprising the helical anti-splicing(+) turf fibers is still largely intact, while the artificial turf 704 comprising the helical anti-splicing(-) turf fibers is severely damaged. The splicing damage has introduced fiber edges that cause diffuse light reflection, resulting in turf 704 appearing white, with a bleached and visibly deteriorated appearance ("whitening"). As can be inferred from the photos in figures 9 and 10, the helical fibers lacking an anti-splicing agent degrade rapidly, the helical fiber gets lost, and the fibers are lying flat or breaking into longitudinal fragments.

**Figure 11** shows a section through an area within a cylindrical extrusion nozzle. This figure shall illustrate the impact of the LDPE anti-splicing agent on the extrusion process and on the mechanical characteristics of the resulting fibers.

"Low-density polyethylene" (LDPE) is a thermoplastic made from the monomer ethylene having a density in the range of 0.910-0.940 g/cm3. Embodiments of the invention are based on LDPE whose density range is within the above specified sub-range.

"Linear low-density polyethylene" (LLDPE) as used herein is a substantially linear polymer (polyethylene), with significant numbers of short branches. LLDPE differs structurally from LDPE because of the absence of long chain branching. The linearity of LLDPE results from the different manufacturing processes of LLDPE and LDPE. In general, LLDPE is produced at lower temperatures and pressures by copolymerization of ethylene and alpha-olefins.

Manufacturing an artificial turf fiber comprising a mixture of LLDPE and LDPE in the amount ranges described may be advantageous for multiple reasons:
The method allows manufacturing artificial turf fibers which are at the same time soft, flexible, resistant to shear forces (e.g. applied during extrusion or during stretching), have a high tensile strength and are resistant to splicing. Applicant has observed that not all plastomers are well suited for preventing splitting in artificial turf fibers, presumably because plastomers - at least if provided in some particular amount ranges and/or having a particular density - appear not to generate a chain entanglement that can reliably prevent splicing and/or have negative side effects like making a fiber that has decreased tensile strength or flexibility and/or an increased brittleness. Applicant has observed that an optimal compromise between a high splicing resistance on the one hand and high tensile strength on the other hand can be achieved by combining specific amounts of LLDPE and LDPE polymers for generating an artificial turf fiber. Said fiber may in addition have a decreased brittleness, decreased splicing risk, and increased flexibility.

Applicant has also observed that the amount of LDPE used should be comparatively low, preferentially in the range of 0.1%-15%, more preferentially in the range of 5-8 % by weight of the polymer mixture to ensure a high resistance to splicing in combination with a high tensile strength and high flexibility of the generated fiber.

Applicant has observed that the lack of long-chain branching in LLDPE allows the chains to slide by one another upon elongation without becoming entangled. As a result, fibers completely consisting of LLDPE are susceptible to splicing if a pulling force is applied on the surface of a fiber. Applicant has also observed that LLDPE has a higher tensile strength and a higher puncture resistance than LDPE and many plastomers.

Applicant has further observed that, upon applying strong shear forces on a polymer mixture comprising LLDPE and LDPE polymers, e.g. by extruding a polymer mixture comprising LLDPE and LDPE polymers, LDPE molecules are deformed, the side branches of the LDPE molecules get entangled with the ones of other LDPE molecules and/or with LLDPE molecules. As a consequence of chain entanglement, the viscosity raises. Applicant found that an artificial turf fiber manufactured from a particular mixture of specific amounts of LDPE and LLDPE is soft and flexible and has high tensile strength (thanks to the LLDPE component) and is at the same time resistant against splicing (thanks to chain entanglement caused by the LDPE component). Applicant has observed that if the ratio of LLDPE to LDPE is too large, splicing may occur, and if said ratio is too low, the flexibility and tensile strength of the fiber may significantly decrease.

Figure 11 shows a section through an area within a cylindrical extrusion nozzle. In a first area 1102, the polymers of a liquefied polymer mixture are mostly in an amorphous state, i.e., there are only few or no crystalline regions and the polymer molecules do not show any preferred orientation in one dimension. In a second area 1104 that corresponds to an area of increased shear forces, the polymer molecules are sheared and pulled at least partially in the direction of the opening 1110 of the nozzle. In the area 1106 corresponding to high shear forces, the LLDPE and partially also the LDPE molecules are at least partially disentangled, oriented and form crystalline portions 1108. However, according to preferred embodiments, the majority of crystalline portions is created later in the stretching process.

Using a LLDPE polymer that comprises also LDPE as an anti-splicing agent, and optionally also a compatibilizer as a further anti-splicing agent, may prevent splicing in artificial turf fibers, because LDPE (in contrast to LLDPE) has side-chains which result in an entanglement of the linear, parallel oriented LLDPE-molecules. The extrusion, and in particular the stretching, results in an at least partial disentanglement and parallel orientation of LLDPE molecules which again causes an increased susceptibility of the fiber to splicing. By adding the appropriate amount of LDPE, in particular LDPE of the preferred density range, to the polymer mixture, the splicing can be prohibited even in helical fibers that are stretched during manufacturing.

**Figure 12** illustrates coextrusion of two polymer components in a coextrusion device for manufacturing an artificial turf fiber with a core-cladding structure. The coextrusion device comprises a joining path 1210 located upstream of a coextrusion opening 1208. The setup comprises a hole 1206 which receives a free end of a capillary tube 1205. Opposite to the inserted capillary tube 1205, hole 1206 ends in coextrusion opening 1208. A clearance between capillary tube 1205 and the walls of hole 1206 hydraulically connects the hole to a second channel system 1204. Capillary tube 1205 is hydraulically connected to a first channel system 1202 and is not fully inserted into hole 1206, such that a section 1210 of hole 1206 is hydraulically connected both to first channel system 1202 and to second channel system 1204. This section 1210 is the joining path 1210 of the depicted coextrusion setup. Joining path 1210 extends from capillary tube 1205 to extrusion opening 1208, as is indicated by dotted lines.

During coextrusion operation, capillary tube 1205 receives a molten core polymer component from first channel system 1202 and hole 1206 receives a molten cladding polymer component from second channel system 1204. The respective transport directions of the polymer components are indicated by arrows. The two polymer components flow separated from each other until they come into contact in joining path 1210. The two joined polymer components pass joining path 1210, which narrows to the cross section of coextrusion opening 1208, and exit coextrusion opening 1208 as a bicomponent monofilament.

In cases where core and cladding are to be joined together with a contact layer comprising a mixture of the core polymer mixture and the cladding polymer component, the dimensions of the joining path are suitably chosen such that a stable contact layer of homogeneous thickness is formed. In an example, the contact layer is formed within an axial length of the joining path of 3 to 7 times the diameter of the liquid core polymer mixture at the upstream end of the joining path. In a more specific example, the diameter of the liquid core polymer mixture at the upstream end of the joining path 1210 is between 0.5 and 1.5 mm, the axial length of the joining path is between 1.5 and 10.5 mm, causing the melted core polymer mixture to mix with the cladding polymer component in a contact layer with a radial thickness between 10 and 150 µm.

The extrusion opening 1208 defines the capillary length 1211 of the extrusion nozzle. The extruding is performed via an extrusion opening, also referred herein as extrusion channel, having a capillary length of less than 2.5 mm, in particular a capillary length of less than 2.0 mm, in particular of 1.4 to 1.6 mm. This may ensure a more turbulent flow, which provides further protection of the helical fiber against splicing.

The capillary length in an extrusion nozzle refers to the length of the narrow, cylindrically shaped passage (capillary) through which the molten material (e.g., polymer) flows as it is forced out of the extrusion die or spinneret. The capillary length determines the shear stress, flow rate, and final properties of the extruded material.

**Figure 13** illustrates different temperature gradients used for generating a helical structure in fibers having different fiber profiles.

For fiber profiles having a maximum length to maximum thickness ratio of less than 3.0, preferably a dorso-ventral temperature gradient is established during the post-processing of the extruded fiber when the fiber is stretched, according to embodiments of the invention.

A dorso-ventral (or "lateral") temperature gradient is a gradient that extends from the lower to the upper surface of the two longitudinal sides of a fiber cross-sectional profile, or from the upper to the lower surface, see the two temperature gradients 1302 and 1304. In general, a dorso-ventral temperature gradient refers to a temperature distribution along the dorsal (back or top) to ventral (top or back) axis, typically seen in non-cylindrical or asymmetrical structures. The temperature difference in this gradient occurs from one side (e.g., dorsal) to the opposite side (e.g., ventral), rather than uniformly radiating outward.

A radially symmetric temperature gradient refers to a temperature distribution that radiates from a central point or axis outward in all directions. In this type of gradient, the temperature changes consistently as one moves away from the center towards the peripheral regions/outer surface.

A dorso-ventral temperature gradient is generated preferably for fibers having a maximum-length to maximum thickness ratio of below 3.0, because the stress introduced via a radial temperature gradient 1306, 1308 has been observed to be in some cases insufficient to outperform the torsion-resistant forces exerted by the massive body of fibers having the above-mentioned length-to-thickness ratio.

However, the dorsoventral temperature gradient typically results in helical fibers which comprise a significant lateral component in their helical turns, meaning that the helical structures generated via a dorso-ventral temperature gradient are wound around a (virtual) cylinder. However, in some cases, it may be more desirable to have a helical fiber that is twisted essentially only around its own axis, i.e., it may be more desirable to have a helical fiber that is twisted like a screw, with basically no lateral component in the helical turns. For those types of applications, and in particular for fibers having a maximum-length to maximum thickness ratio of above 3.0, or above 4.0, or above 5.0, and in particular for fiber profiles having a central bulb and two wings extending in different directions, a radial temperature gradient is preferably established according to embodiments. For example, the temperature gradients 1306 and 1308, which are both examples for radial temperature gradients from the central part to the peripheral regions of the fiber profile cross section, are used for causing fiber profiles 504-510 of figure 5 to acquire helical shape. Applicant has observed that the helical shape caused by the radial temperature gradients 1306, 1308 and a stretching or compression of the fibers when this temperature gradient is present results in helical structures which have no or only a small lateral component in their helical rotations, meaning they look more like screws and less like helices coiled around a virtual cylinder. Applicant has observed, however, that a radial temperature gradient 1306, 1308 is less suitable for creating a helical structure in fibers with a maximum length to maximum thickness ratio of less than 3.0. Rather, a radial temperature gradient is preferably established for fiber profiles having a ratio of 3.0 or higher, e.g., 4.0 or higher, or 5.0 or higher.

According to embodiments, the dorso-ventral temperature gradients and he radial temperature gradients are combined to generate fibers being strongly twisted around their own axis and having in addition a lateral rotational movement around a virtual cylinder.

According to embodiments, the temperature gradient 1302, 1304, 1306, 1308 established across the fiber cross section has a temperature difference between the hottest and coldest temperature in the gradient of at least 10°C, preferably of at least 20°C.

For example, the fiber leaving the extrusion nozzle may have a temperature of about 240°, and the fiber may be quenched in a water bath of about 25°C. In some embodiments, the fiber may already be stretched during the quenching, so a radial temperature gradient of a freshly extruded fiber that has just entered the quenching bath may range from about 240°C at the central core of the fiber to about 25°C at the fiber surface.

According to other examples, the fiber may be transported via a series of cooled and heated godets, wherein the cooled godets are contacting the lower surface of the fiber while the heated godets are contacting the upper surface of the fiber. The rotation speed of these godets may be different, thereby stretching the fiber during the transport. The "lower" and "upper" surfaces of the fiber refer to the two surfaces having approximately the same orientation as the longitudinal axis of the fiber profile. The cooled godets may have, for example, a temperature of about 15°C, and the heated godets may have a temperature of about 50°C, and the resulting dorsoventral temperature gradient may range from about 15°C at the lower fiber surface to about 50°C at the upper surface.

A 'temperature gradient across a fiber cross section' as used herein refers to the variation or difference in temperature between different points within the cross-sectional area of a fiber. It describes how the temperature changes from one location to another, e.g., from the core (center) of the fiber to the outer surface, or from a lower fiber surface to the upper fiber surface, or vice versa. The temperature gradient may be linear or non-linear.

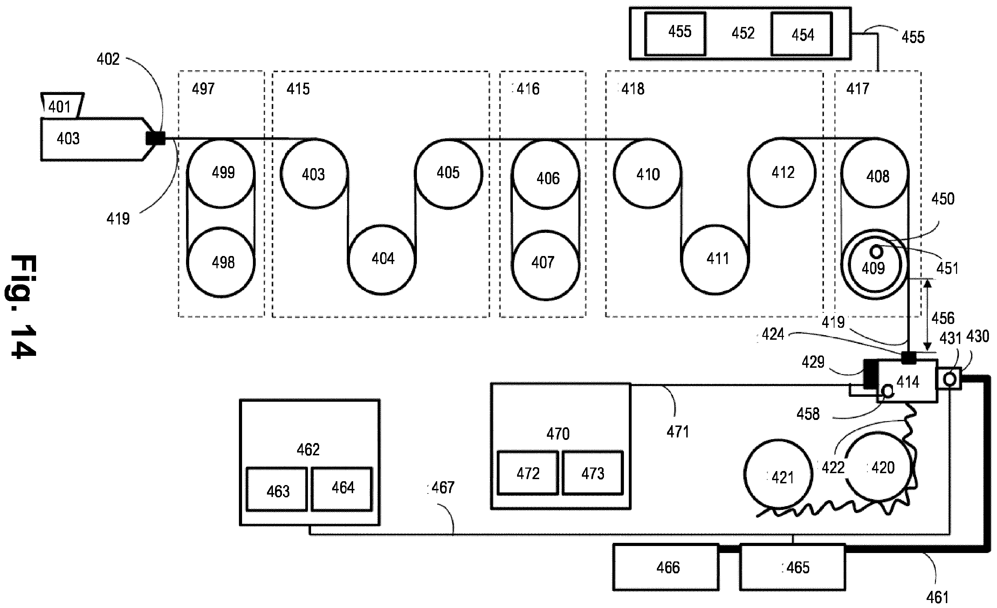

**Figure 14** is a block diagram of a manufacturing line for manufacturing artificial turf yarn 422. The manufacturing line (or manufacturing system) comprises: an extruder 403 (e.g. a screw-extruder) , one or more drawing devices 415, 418, one or more thermosetting (or heating) devices (e.g. godets, ovens) 417, one or more cooling devices (e.g. godets, bathes with cooling liquid) 416, 420, 497, and one or more rollers 421.

The extruder 403 comprises at least one hopper 401 for feeding components of a monofilament yarn (e.g. a blend of polymers) into the extruder and one outlet 402 for the monofilament yarn. The outlet 402 can be implemented as a wide slot nozzle or a spinneret. A polymer melt formed in a chamber of the extruder is pressed through the outlet 402 to form a monofilament yarn of a specific shape.

The monofilament yarn can be cooled down after the extrusion using the cooling device 497. When the cooling device is implemented as a godet, it can comprise two rollers 499 and 498 for winding the monofilament yarn 419. The cooling process can be implementing by maintaining a temperature of the rollers 499 and 498 within the specified range and/or by air cooling and/or by water cooling. A temperature of water (or air) can be kept within a specified range as well. Alternatively, the cooling device can be a bath with a cooling liquid (e.g. water) in which the monofilament yarn is cooled. The monofilament yarn is cooled down using the cooling device 497 to a temperature where crystallization can take place. The cooling device 497 may be, for example, a water bath (quenching bath). In the crystallization process the crystallites are forming to a percentage, which depends on the cooling rate. The higher the cooling rate, the less is the crystallinity and vice versa. The temperature of one or more of these devices, the environment temperature and/or the speed of transportation of the fiber from one processing unit to the next may be chosen such that a stable temperature gradient is reproducibly established across the cross section of the fiber.

The monofilament yarn can be further drawn using the drawing device 415. The drawing device can comprise three rollers 404, 403, 405. The drawing ratio is defined as the ratio of linear speeds of a pair of rollers 403 and 404 (or 404 and 405). The drawing device 415 can be operable for heating the monofilament yarn 419 during or before the drawing process such that a temperature gradient is established in the cross-sectional profile of the fiber. This can be implemented by differentially heating, e.g., heating and/or cooling one or more the rollers in order to keep their temperature within a predetermined temperature range and/or by air heating, wherein the hot air has a temperature within a predetermined temperature range, whereby the hot air is applied selectively to one side of the fiber. The elongation of the monofilament yarn in the drawing device can force the macromolecules of the monofilament yarn to parallelize and will introduce stress in the polymer material, because the molecules will reorganize in response to the mechanical stretching forces in a temperature-dependent way and the temperature gradient results in different molecular re-organization in different parts of the fiber cross section. This results in an overall higher degree of crystallinity and increased tensile strength, compared with undrawn monofilament yarn, but the crystallinity is not uniformly distributed in the fiber cross-section.

According to an alternative example not depicted in a figure, the drawing device may comprise one or more feeding rollers, an oven, and one or more receiving rollers. The one or more feeding rollers are configured to feed the monofilament yarn 419 into the oven. The one or more receiving rollers are configured to receive the monofilament yarn from the oven. The oven is configured to heat the monofilament yarn. The drawing ratio is determined by a ratio of the linear speeds of the feeding roller being the last roller before the oven and the receiving roller being the first after oven. The thermosetting process (drawing process) is performed in the oven 480, in which the monofilament yarn in stretched and heated simultaneously.

The monofilament yarn can be further cooled using the cooling device 416. The cooling device, when implemented as a cooling godet can have rollers 406 and 407. Alternatively, the cooling device 416 can be built and/or function in the same way as the cooling device 497. Afterwards the monofilament yarn can be further drawn using the drawing device 418 having rollers 410, 411, and 412. The drawing device 118 can be built and/or function in the same way as the drawing device 415.

The monofilament yarn can be further heated using one or more heating devices or elements (e.g. device 417). The heating device comprises a heater (or a heating element) and a temperature sensor for sensing a temperature of the heater (or the heating element). The heater can be implemented as an electrical resistance heater. The heating device is controlled by a controller (e.g. controller 452) such that the temperature of the heater is kept at a desired temperature (this temperature is mentioned herein as the third desired temperature as well). The controller comprises a computer processor 453 and memory 454 comprising instructions executable by the computer processor. The controller is communicatively coupled to the heating device and the temperature sensor configured to sense a temperature of the heating device. The communicative coupling can be implemented via a computer network 455.

The controller is operable to hold an actual temperature of the heating device at the desired temperature. The desired temperature can be selected such that a stable temperature gradient is established in the cross-section of the yarn during a transportation from the heater to the texturing apparatus (e.g. distance 456) has a temperature of the texturing process when it enters the texturing apparatus 414, or its inlet port 424 for receiving the yarn. The execution of the computer instructions by the computer processor 453 causes the controller to maintain the desired temperature gradient.

The heating device 417, when implemented as a godet, comprises a pair of rollers 408 and 409. The heating of the monofilament yarn can be made by keeping a temperature of the rollers within a predetermined temperature range and/or by hot air having a temperature within a predetermined temperature range. For instance the roller 409 can be equipped with a heater 150 and a temperature sensor 451 both communicatively coupled to the controller 452.

A controller 470 comprises a computer processor 472 and memory 473 comprising instructions executable by the computer processor.

The controller may optionally be configured to control a temperature of an optional texturing apparatus 414. The controller is communicatively coupled to the temperature sensor 458 configured to sense a temperature of the texturing apparatus 414, and a heating device, 429. The heating device can be configured to heat the texturing device through physical contact between the texturing device and the heating device or by electromagnetic induction. The physical contact can be a direct physical contact or a contact in which a thermally conductive paste is used between the heating device 429 and the texturing apparatus 414. At least a portion of the texturing device can be placed inside or in the proximity of the electromagnet of the heating device configured to heat the texturing device by electromagnetic induction. The heating device can be implemented as an electrical resistance heater. Further heating devices and temperature sensors can be operated by the controller 470 (or other controllers). The communicative coupling can be implemented via a computer network 471. The controller is operable to hold an actual temperature of the texturing apparatus at a desired temperature which can be the temperature required for the texturing process. The desired temperature can be specified as a temperature range. In this case the holding of the actual temperature at the desired temperature comprises keeping the actual temperature within the specified range, in particular the actual temperature is kept as close as possible to a middle temperature of the temperature range. The middle temperature is equal to an average of a lower boundary of the temperature range and an upper boundary of the chosen temperature range. The execution of the computer instructions by the computer processor 472 causes the controller to hold the texturing apparatus temperature at the desired temperature.

After the heating using one or more heating devices 417 the monofilament yarn is textured (curled) in the texturing apparatus 414. The textured (curled) monofilament yarn 422 is cooled using a cooling godet 420. And is forwarded further to another roller 421 for further processing.

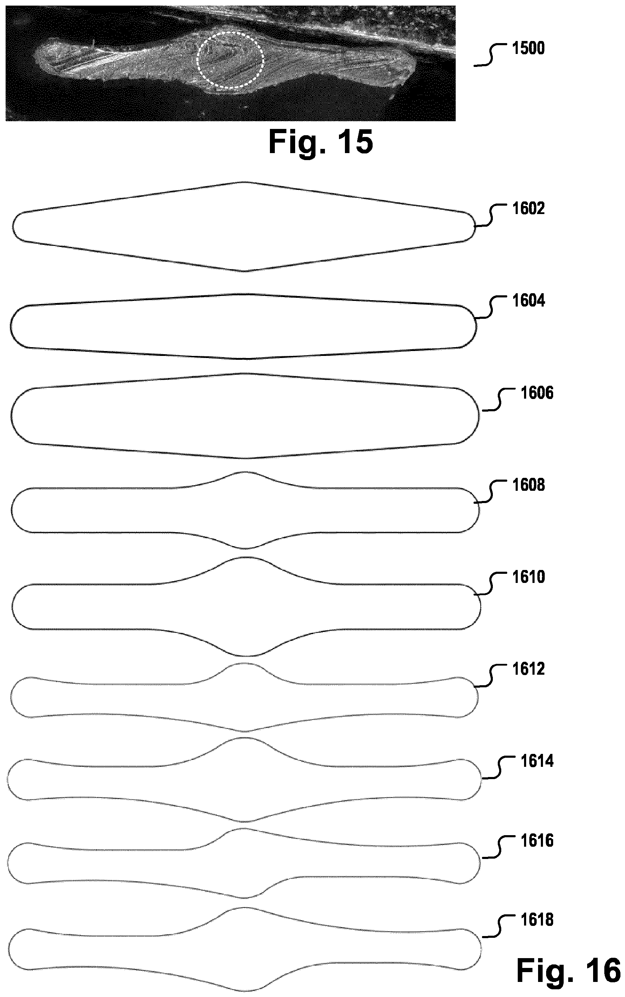

**Figure 15** is a photo of a cross-section of a short segment of a longitudinal, helical fiber 1500. The depicted fiber is made of only a single PE-based polymer. According to some embodiments, the area within the dotted ring of figure 15 may represent a central region of the cross-sectional profile of the fiber 1500 and the area outside of the dotted ring of the fiber 1500 may represent the peripheral region of the cross-sectional fiber profile. The periphery of the artificial turf fiber may comprise the two wings and a thin layer within the central bulb that surrounds the core polymer. The central region may consist of a core polymer, and the peripheral region may consist of a cladding polymer (also referred to as wing polymer).

**Figure 16** shows various examples of cross-sectional profiles of artificial turf fibers 1602-1618 according to embodiments of the invention. The artificial turf fibers 1602-1618 have a longitudinal cross section, meaning the longest cross-sectional diameter of the fiber is at least twice the length of the shortest cross-sectional diameter. According to some embodiments, the fiber comprises a central bulb, which may be only slightly thicker than the wings of the fiber (as shown e.g. in 1602-1606) or which may be significantly, e.g., at least 10%, or at least 15%, or at least 20% thicker than the wings (measured at the thinnest portion of any of the two wings). For example, the central bulb in fiber profiles 1608-1618 is significantly thicker than the wings. Preferably, the fiber profiles comprise a core polymer at the center of the central bulb, and a cladding polymer at the periphery of the artificial turf fiber. According to some embodiments, the fiber profile is axially symmetrical with respect to the x and y axis (see e.g. fiber profiles 1602-1610). According to some embodiments, the fiber profile is rotational symmetric (see profiles 1616 and 1618).

Disclosed herein are also the following fibers and methods for manufacturing the same, which are described as clauses which can freely be combined with each other and with the other examples and embodiments disclosed herein as long as they are not mutually exclusive:

Clauses related to fibers comprising an anti-splicing agent:
1. An artificial turf fiber (204, 502-510, 804, 902-906) comprising an anti-splicing agent, the artificial turf fiber being under internal stress that causes the artificial turf fiber, upon receiving heat treatment, to assume a helical shape.
2. A helical artificial turf fiber, comprising an anti-splicing agent.
3. The artificial turf fiber of any one of the previous clauses, wherein the helical artificial turf fiber has a 3D-helical shape characterized in that the fiber winds around a central cylinder, wherein the fiber maintains a distance from the cylinder axis as it rotates, and advances along the axis, resulting in a three-dimensional spiral curve.
4. The artificial turf fiber of any one of the preceding clauses, wherein the helical artificial turf fiber is a twisted fiber that is rotated around its own axis.
5. The artificial turf fiber according to any one of the preceding clauses, wherein the anti-splicing agent is or comprises a compatibilizer.
6. The artificial turf fiber according to clause 5, wherein the compatibilizer is selected from a group comprising: an anhydride modified polyethylene, a maleic acid grafted on polyethylene or polyamide; a maleic anhydride grafted on free radical initiated graft copolymer of polyethylene, SEBS, EVA, EPD, or polypropylene with an unsaturated acid or it's an- hydride such as maleic acid, glycidyl methacrylate, ricinoloxazoline maleinate; a graft copolymer of SEBS with glycidyl methacrylate, a graft copolymer of EVA with mercaptoacetic acid and maleic anhydride; a graft copolymer of EPDM with maleic anhydride; Ethylene Ethyl Acrylate (EEA), Maleic Acide Anhydride grafted PE, a graft copolymer of polypropylene with maleic anhydride; a polyolefin-graftpolyamidepolyethylene or polyamide; and a polyacrylic acid type compatibilizer; and a combination of two or more of the aforementioned substances,
   wherein the compatibilizer is in particular Ethylene Ethyl Acrylate (EEA) or Maleic Acide Anhydride grafted PE.
7. The artificial turf of clause 5 or 6, the fiber comprising the compatibilizer in an amount of 0.5- 5.0 weight %, in particular 0.5- 2.0 weight %, wherein in particular the compatibilizer is EEA or Maleic Acid Anhydride grafted PE.
8. The artificial turf fiber according to any of the preceding clauses, wherein the anti-splicing agent is or comprises a low-density polyethylene - LDPE.
9. The artificial turf fiber according to clause 8, wherein the fiber comprises the LDPE polymer in an amount of 0.1-15 % by weight of the fiber, in particular in an amount of 5-8 % by weight of the fiber.
10. The artificial turf fiber of any one of the preceding clauses, wherein the artificial turf fiber has a cross-sectional profile with a central bulb and two wings extending in different directions.
11. The artificial turf fiber according to any one of the preceding clauses, wherein the fiber comprises a different polymer material at its core compared to its peripheral regions, wherein the polymer materials are selected such that the polymer material at the core exhibits greater shrinkage than the polymer material in the peripheral regions in response to heat treatment.
12. The artificial turf fiber according to any one of the preceding clauses, comprising:
   - 0.1-15%, in particular 5.0-8.0% by weight of low-density polyethylene (LDPE), and
   - optionally 0.1-15% by weight HDPE; and
   - 60-99% by weight of linear low-density polyethylene (LLDPE).
13. The artificial turf fiber according to any one of the preceding clauses, wherein at least 60% by weight of the fiber, preferably at least 75% by weight of the fiber, comprises one or more polymers having a density in the range of 0.910 to 0.928 g/cm³, more preferably in the range of 0.913 to 0.925 g/cm³.
14. The artificial turf fiber according to any one of the preceding clauses, wherein the fiber comprises an hydrophilization agent, in particular hydrophilic fumed silica.
15. The artificial turf fiber according to any one of the preceding clauses, wherein the fiber comprises a nucleating agent.
16. An artificial turf, comprising:
   - a carrier
   - a plurality of helical artificial turf fibers according to any one of the previous clauses 2-15, wherein the fibers are integrated into the carrier and extend to one side of the carrier.
17. The artificial turf according to clause 16,
   - wherein the helical artificial turf fibers are texturized fibers,
   - wherein the artificial turf further comprises non-texturized fibers, wherein in particular the non-texturized fibers have approximately the same length or a larger length than the helical, texturized fibers.
18. The artificial turf according to clause 16 or 17, further comprising:
   - an infill layer,
   - wherein the number of twists per fiber and the height of the infill is chosen such that in at least 80% of the helical fibers, the part of the helical fibers that protrudes above the infill layer makes at least 1.0 turns.
19. A method for manufacturing an artificial turf fiber that is under internal stress, the method comprising:
   - creating (102) a polymer mixture, the mixture comprising an anti-splicing agent:
   - extruding (104) the polymer mixture into a monofilament;
   - quenching (106) the monofilament;
   - controlling (108) equipment for processing the monofilament such that a temperature gradient is formed reproducibly in the cross-section of the monofilament;
   - stretching (110) the monofilament while the temperature gradient is present to form the monofilament into the artificial turf fiber, thereby introducing the internal stress into the fiber, wherein the internal stress is adapted to cause the artificial turf fiber, upon receiving heat treatment, to assume a helical shape.
20. A method for manufacturing a helical artificial turf fiber, comprising:
   - creating (102) a polymer mixture, the mixture comprising an anti-splicing agent:
   - extruding (104) the polymer mixture into a monofilament;
   - quenching (106) the monofilament;
   - controlling (108) equipment for processing the monofilament such that a temperature gradient is formed reproducibly in the cross-section of the monofilament;
   - stretching (110) the monofilament while the temperature gradient is present to form the monofilament into the artificial turf fiber, thereby introducing stress into the fiber; and
   - heating (112) the monofilament, thereby causing the artificial turf fiber change its 3D shape to form a helical artificial turf fiber.
21. The method according to clauses 19 or 20, wherein the extruding is performed via an extrusion channel having a capillary length of less than 2.5 mm, in particular a capillary length of less than 2.0 mm, in particular of 1.4 to 1.6 mm.
22. The method according to clauses 19, 20 or 21, wherein the stretching (110) is performed such that the ratio of the fiber length before the stretching to the fiber length after the stretching is in the range of 1:4.0 to 1:5.1.

Clauses related to fibers comprising a hydrophilization agent
1. An artificial turf fiber (204, 502-510, 804, 902-906) comprising an hydrophilization agent, the artificial turf fiber being under internal stress that causes the artificial turf fiber, upon receiving heat treatment, to assume a helical shape.
2. A helical artificial turf fiber, comprising a hydrophilization agent.
3. The artificial turf fiber of any one of the previous clauses, wherein the helical artificial turf fiber has a 3D-helical shape characterized in that the fiber winds around a central cylinder, wherein the fiber maintains a distance from the cylinder axis as it rotates, and advances along the axis, resulting in a three-dimensional spiral curve.
4. The artificial turf fiber of any one of the preceding clauses, wherein the helical artificial turf fiber is a twisted fiber that is rotated around its own axis.
5. The artificial turf fiber according to any one of the preceding clauses, wherein the hydrophilization agent is or comprises hydrophilic fumed silica.
6. The artificial turf fiber according to any one of the preceding clauses,
   - wherein the hydrophilization agent is or comprises a surfactant, in particular polyenthylene glycol - PEG, and/or
   - wherein the hydrophilization agent is or comprises Maleic Acide Anhydride grafted PE.
7. The artificial turf fiber according to any one of the preceding clauses, wherein the fiber comprises a nucleating agent.
8. The artificial turf fiber according to clause 7, wherein the nucleating agent is an inorganic and/or an organic substance or a mixture thereof,
   wherein the inorganic substance acting as the nucleating agent consists of one or more of the following:
      - talcum;
      - kaolin;
      - calcium carbonate;
      - magnesium carbonate;
      - silicate;
      - silicic acid;
      - silicic acid ester;
      - aluminium trihydrate;
      - magnesium hydroxide;
      - meta- and/or polyphosphates; and
      - coal fly ash;
      - fumed silica;
   wherein the organic substance acting as the nucleating agent consists of one or more of the following:
      - 1,2-cyclohexane dicarbonic acid salt;
      - benzoic acid;
      - benzoic acid salt;
      - sorbic acid; and
      - sorbic acid salt.
9. The artificial turf fiber according to any one of the preceding clauses, wherein the artificial turf further comprises an anti-splicing agent.
10. The artificial turf fiber according to clause 9, wherein the anti-splicing agent is or comprises a compatibilizer.
11. The artificial turf fiber according to clause 10, wherein the compatibilizer is selected from a group comprising: an anhydride modified polyethylene, a maleic acid grafted on polyethylene or polyamide; a maleic anhydride grafted on free radical initiated graft copolymer of polyethylene, SEBS, EVA, EPD, or polypropylene with an unsaturated acid or it's an- hydride such as maleic acid, glycidyl methacrylate, ricinoloxazoline maleinate; a graft copolymer of SEBS with glycidyl methacrylate, a graft copolymer of EVA with mercaptoacetic acid and maleic anhydride; a graft copolymer of EPDM with maleic anhydride; Ethylene Ethyl Acrylate (EEA), Maleic Acide Anhydride grafted PE, a graft copolymer of polypropylene with maleic anhydride; a polyolefin-graftpolyamidepolyethylene or polyamide; and a polyacrylic acid type compatibilizer; and a combination of two or more of the aforementioned substances,
   wherein the compatibilizer is in particular Ethylene Ethyl Acrylate (EEA) or Maleic Acide Anhydride grafted PE.
12. The artificial turf of clause 10 or 11, the fiber comprising the compatibilizer in an amount of 0.5- 5.0 weight %, in particular 0.5- 2.0 weight %, wherein in particular the compatibilizer is EEA or Maleic Acid Anhydride grafted PE.
13. The artificial turf fiber according to any of the preceding clauses, wherein the anti-splicing agent is or comprises a low-density polyethylene - LDPE.
14. The artificial turf fiber according to clause 13, wherein the fiber comprises the LDPE polymer in an amount of 0.1-15 % by weight of the fiber, in particular in an amount of 5-8 % by weight of the fiber.
15. The artificial turf fiber of any one of the preceding clauses, wherein the artificial turf fiber has a cross-sectional profile with a central bulb and two wings extending in different directions.
16. The artificial turf fiber according to any one of the preceding clauses, wherein the fiber comprises a different polymer material at its core compared to its peripheral regions, wherein the polymer materials are selected such that the polymer material at the core exhibits greater shrinkage than the polymer material in the peripheral regions in response to heat treatment.
17. The artificial turf fiber according to any one of the preceding clauses, comprising:
   - 0.1-15%, in particular 5.0-8.0% by weight of low-density polyethylene (LDPE), and
   - optionally 0.1-15% by weight HDPE; and
   - 60-99% by weight of linear low-density polyethylene (LLDPE).
18. The artificial turf fiber according to any one of the preceding clauses, wherein at least 60% by weight of the fiber, preferably at least 75% by weight of the fiber, comprises one or more polymers having a density in the range of 0.910 to 0.928 g/cm³, more preferably in the range of 0.913 to 0.925 g/cm³.
19. An artificial turf, comprising:
   - a carrier
   - a plurality of helical artificial turf fibers according to any one of the previous clauses 2-18, wherein the fibers are integrated into the carrier and extend to one side of the carrier.
20. The artificial turf according to clause 19,
   - wherein the helical artificial turf fibers are texturized fibers,
   - wherein the artificial turf further comprises non-texturized fibers, wherein in particular the non-texturized fibers have approximately the same length or a larger length than the helical, texturized fibers.
21. The artificial turf according to clause 19 or 20, further comprising:
   - an infill layer,
   - wherein the number of twists per fiber and the height of the infill is chosen such that in at least 80% of the helical fibers, the part of the helical fibers that protrudes above the infill layer makes at least 1.0 turns.
22. A method for manufacturing an artificial turf fiber that is under internal stress, the method comprising:
   - creating (102) a polymer mixture, the mixture comprising a hydrophilization agent:
      - extruding (104) the polymer mixture into a monofilament;
      - quenching (106) the monofilament;
      - controlling (108) equipment for processing the monofilament such that a temperature gradient is formed reproducibly in the cross-section of the monofilament;
      - stretching (110) the monofilament while the temperature gradient is present to form the monofilament into the artificial turf fiber, thereby introducing the internal stress into the fiber, wherein the internal stress is adapted to cause the artificial turf fiber, upon receiving heat treatment, to assume a helical shape.
23. A method for manufacturing a helical artificial turf fiber, comprising:
   - creating (102) a polymer mixture, the mixture comprising a hydrophilization agent:
      - extruding (104) the polymer mixture into a monofilament;
      - quenching (106) the monofilament;
      - controlling (108) equipment for processing the monofilament such that a temperature gradient is formed reproducibly in the cross-section of the monofilament;
      - stretching (110) the reheated monofilament while the temperature gradient is present to form the monofilament into the artificial turf fiber, thereby introducing stress into the fiber; and
      - heating (112) the monofilament, thereby causing the artificial turf fiber change its 3D shape to form a helical artificial turf fiber.
24. The method according to clauses 22 or 23, wherein the extruding is performed via an extrusion channel having a capillary length of less than 2.5 mm, in particular a capillary length of less than 2.0 mm, in particular of 1.4 to 1.6 mm.
25. The method according to clauses 22, 23 or 24, wherein the stretching (110) is performed such that the ratio of the fiber length before the stretching to the fiber length after the stretching is in the range of 1:4.0 to 1:5.1.
26. The method according to any one of clauses 22-25, wherein the temperature gradient is a dorso-ventral gradient or a radial gradient.
27. The method according to clause 26, wherein the fiber has a maximum-length-to-maximum-thickness ratio below 3.0 and wherein the temperature gradient is a dorsoventral gradient.
28. The method according to clause 26, wherein the fiber has a maximum-length-to-maximum-thickness ratio of at least 3.0, in particular at least 4.0, in particular at least 5.0, and wherein the temperature gradient is a radial gradient.
29. The method according to any one of clauses 22-28,
   - wherein the manufacturing equipment comprises godets; and
   - wherein the controlling (108) of the equipment for generating the temperature gradient comprises: controlling the temperature of the godets or parts thereof such that a temperature gradient within the fiber cross-section is reproducibly introduced into the material of the fiber.
30. The method of clause 29, wherein the stretching of the fiber comprises stretching the fiber by the godets while transporting the fiber.
31. The method according to any one of clauses 22-30,
   - wherein the manufacturing equipment comprises a stretching chamber; and
   - wherein the controlling (108) of the equipment for generating the temperature gradient comprises: controlling the temperature of the stretching chamber or parts thereof such that a temperature gradient within the fiber cross-section is reproducibly introduced into the material of the fiber.
32. The method of clause 31, wherein the stretching chamber comprises at least one heating element, and wherein the introduction of the temperature gradient within the fiber cross-section comprises: controlling the heating element in the stretching chamber so that a temperature gradient within the fiber cross-section is reproducibly introduced into the material of the fiber.

Clauses related to fibers comprising a longitudinal cross-sectional shape with different polymers:
1. An artificial turf fiber (204, 502-510, 804, 902-906, 1500, 1602-1618) comprising a longitudinal cross-sectional profile, the artificial turf fiber being under internal stress that causes the artificial turf fiber, upon receiving heat treatment, to assume a helical shape, wherein the fiber comprises a core polymer in its core and a cladding polymer its periphery, wherein the core polymer and the cladding polymer are different polymer materials, and wherein the core and cladding polymers are selected such that the core polymer undergoes greater shrinkage than the cladding polymer when subjected to the heat treatment.
2. A helical artificial turf fiber (204, 502-510, 804, 902-906, 1500, 1602-1618) comprising a longitudinal cross-sectional profile, wherein the fiber comprises wherein the fiber comprises a core polymer in its core and a cladding polymer its periphery, wherein the core polymer and the cladding polymer are different polymer materials, and wherein the core and cladding polymers are selected such that the core polymer undergoes greater shrinkage than the cladding polymer when subjected to the heat treatment.
3. The artificial turf fiber according to clause 1 or 2, wherein the cross-sectional profile has a maximum length to maximum thickness ratio of at least 3.0, in particular at least 4.0, in particular at least 5.0,
4. The artificial turf fiber according to any one of the previous clauses, wherein the cross-sectional fiber profile has a central bulb and two wings extending from the central bulb in different directions.
5. The artificial turf fiber according to clause 4, wherein the core polymer is comprised in the core of the central bulb and wherein the cladding polymer is comprised in the wings and optionally also in peripheral regions of the central bulb.
6. The artificial turf fiber of any one of the previous clauses, wherein the helical artificial turf fiber has a 3D-helical shape characterized in that the fiber winds around a central cylinder, wherein the fiber maintains a distance from the cylinder axis as it rotates, and advances along the axis, resulting in a three-dimensional spiral curve.
7. The artificial turf fiber of any one of the preceding clauses, wherein the helical artificial turf fiber is a twisted fiber that is rotated around its own axis.
8. The artificial turf fiber according to any one of the preceding clauses, wherein the cross-sectional profile has a maximum length to maximum thickness ratio of below 3.0, and wherein the helical artificial turf fiber has a 3D-helical shape characterized in that the fiber winds around a central cylinder, wherein the fiber maintains a distance from the cylinder axis as it rotates, and advances along the axis, resulting in a three-dimensional spiral curve.
9. The artificial turf fiber according to any one of the clauses 1-7, wherein the cross-sectional profile has a maximum length to maximum thickness ratio of at least 3.0, in particular at least 4.0, in particular at least 5.0, and wherein the helical artificial turf fiber is a twisted fiber that is rotated around its own axis.
10. The artificial turf fiber according to clause 9, wherein the helical fiber does not comprise a lateral component as it rotates and advances along its own central axis, resulting in a three-dimensional spiral curve having the shape of a straight screw.
11. The artificial turf fiber according to any one of the preceding clauses, wherein the artificial turf has a core-cladding structure as the result of a co-extrusion step of a stream of the core polymer concentrically surrounded by a stream of the cladding-polymer.
12. The artificial turf fiber according to any one of the preceding clauses, wherein the core polymer is or predominantly comprises polyethylene and the cladding polymer is or predominantly comprises polyamide.
13. The artificial turf fiber according to any one of clauses, wherein the core polymer is a blend of aged polymer and a first virgin polymer, wherein the cladding polymer is made of a second virgin polymer, and wherein in particular the first and the second virgin polymers are both polyethylene polymers.
14. The artificial turf fiber according to any one of the previous clauses 8-10, wherein the cladding polymer comprises a higher amount of HDPE than the core polymer.
15. The artificial turf fiber according to any one of the preceding clauses, wherein the artificial turf comprises an anti-splicing agent.
16. The artificial turf fiber according to clause 15, wherein the anti-splicing agent is a compatibilizer, and/or a low-density polyethylene - LDPE.
17. The artificial turf fiber according to any one of the preceding clauses, wherein the fiber comprises an hydrophilization agent, in particular hydrophilic fumed silica.
18. The artificial turf fiber according to any one of the preceding clauses, wherein the fiber comprises a nucleating agent.
19. An artificial turf, comprising:
   - a carrier
   - a plurality of helical artificial turf fibers according to any one of the previous clauses 2-18, wherein the fibers are integrated into the carrier and extend to one side of the carrier.
20. The artificial turf according to clause 19,
   - wherein the helical artificial turf fibers are texturized fibers,
   - wherein the artificial turf further comprises non-texturized fibers, wherein in particular the non-texturized fibers have approximately the same length or a larger length than the helical, texturized fibers.
21. The artificial turf according to clause 19 or 20, further comprising:
   - an infill layer,
   - wherein the number of twists per fiber and the height of the infill is chosen such that in at least 80% of the helical fibers, the part of the helical fibers that protrudes above the infill layer makes at least 1.0 turns.
22. A method for manufacturing an artificial turf fiber that is under internal stress, the method comprising:
   - extruding (104) a combination of a core polymer and a cladding polymer into a monofilament via an extrusion nozzle causing the monofilament to have a longitudinal cross-sectional profile, in particular a cross-sectional profile with a central bulb and two wings extending from the central bulb in different directions, wherein the core and cladding polymers are selected such that the core polymer undergoes greater shrinkage than the cladding polymer when subjected to the heat treatment, wherein the extrusion is performed such that the core polymer is exclusively or predominantly located in the core of the cross-sectional fiber profile and wherein the cladding polymer is exclusively or predominantly located in peripheral regions of the cross-sectional profile;
   - quenching (106) the monofilament;
   - controlling (108) equipment for processing the monofilament such that a temperature gradient is formed reproducibly in the cross-section of the monofilament;
   - stretching (110) the monofilament while the temperature gradient is present to form the monofilament into the artificial turf fiber, thereby introducing the internal stress into the fiber, wherein the internal stress is adapted to cause the artificial turf fiber, upon receiving heat treatment, to assume a helical shape.
23. The method of claim 22, further comprising:
   - heating (112) the stretched monofilament, thereby causing the artificial turf fiber to assume a helical shape and become a helical artificial turf fiber.
24. The method according to clauses 22 or 23, further comprising creating (102) at least one polymer mixture comprising at least the core polymer and the cladding polymer. For example, the combination of the core and the cladding polymer may be a combination of two separate polymer mixtures which are fed separately into a co-extrusion nozzle, wherein a first one of the two mixtures may comprise the core polymer and one or more optional additives, and a second one of the mixtures may comprise the cladding polymer and one or more optional additives. In case a single-nozzle-extrusion approach is used, the combination of the core and cladding polymer may be provided in the form of a single polymer mixture (a polymer blend) which is fed into the extrusion nozzle.
   For example, the extruding may be performed via an extrusion channel having a capillary length of less than 2.5 mm, in particular a capillary length of less than 2.0 mm, in particular of 1.4 to 1.6 mm.
25. The method according to clauses 22, 23 or 24, wherein the stretching (110) is performed such that the ratio of the fiber length before the stretching to the fiber length after the stretching is in the range of 1:4.0 to 1:5.1.
26. The method according to clauses 22-25, wherein the extrusion nozzle is a coextrusion nozzle for co-extruding a core polymer and a cladding polymer, wherein the core polymer is or comprises the first polymer, wherein the cladding polymer is or comprises the second polymer, wherein the central bulb is made of the core polymer and a layer of cladding polymer surrounding the core polymer, wherein the wings are made of the cladding polymer. The first polymer is a polymer that shrinks stronger upon heat treatment than the second polymer.
27. The method according to clauses 22-26, wherein the method comprises:
   - Generating a three-phase polymer mixture, the polymer mixture comprising mainly a first polymer, in particular PA, comprising a second polymer, in particular PE, and a compatibilizer, wherein the second polymer forms beads within the first polymer, the beads being surrounded by the compatibilizer, wherein the three phase polymer mixture is used as the extruded at least one mixture; and
   - Performing the extrusion, wherein the polymer beads are located predominantly in the core during extrusion, wherein the polymer beads are deformed into thread-like regions in the extrusion process, wherein the thread-like regions of the polymer are located mainly in the core, and where in the core and undergoes greater shrinkage upon being subject to heat-treatment than the periphery of the cross-sectional profile.
28. The method of any one of the previous clauses 22-27, wherein the cross-sectional profile has a maximum length to maximum thickness ratio of below 3.0, and wherein the equipment is controlled such that a dorso-ventral temperature gradient is formed.
29. The method of any one of the previous clauses 22-27, wherein the cross-sectional profile has a maximum length to maximum thickness ratio of at least 3.0, in particular at least 4.0, in particular at least 5.0, and wherein the equipment is controlled such that a radial temperature gradient is formed.

### REFERENCE SIGNS LIST

102-110 steps
200 artificial turf
202 carrier
204 texturized and helical fiber
206 straight fiber
208 infill
300 artificial turf
302 height of layer of texturized, helical fibers
304 height of straight fibers
400 artificial turf
401 Hopper
402 Outlet
403 Extruder
404 Roller
405 Roller
406 Roller
407 Roller
408 Roller
409 Roller
410 Roller
411 Roller
412 Roller
414 Texturing apparatus
415 Drawing device
416 Cooling device
417 Heating device
418 Drawing device
419 Monofilament yarn
420 Cooling godet
421 Roller
422 Artificial turf yarn
424 Inlet port
429 Heating device
451 Temperature sensor
452 Controller
453 Computer processor
454 Memory
455 Computer network
456 Distance (from heater to texturing apparatus)
458 Temperature sensor (texturing apparatus)
470 Controller
472 Computer processor
473 Memory
480 Oven (thermosetting process)
497 Cooling device
498 Roller
499 Roller
502-510 artificial turf fiber profile
600 Lisport Test apparatus
700 area covered with two different types of artificial turf
702 artificial turf with artificial turf fibers with an anti-splicing agent
704 artificial turf with artificial turf fibers without an anti-splicing agent
800 artificial turf with artificial turf fibers with an anti-splicing agent
802 artificial turf with artificial turf fibers without an anti-splicing agent
804 artificial turf with fiber with an anti-splicing agent
806 artificial turf fiber without an anti-splicing agent
902-912 photos of fibers
1102 first area
1104 second area
1106 area 1106 corresponding to high shear forces
1108 crystalline portions
1110 nozzle opening
1202 First channel system
1204 Second channel system
1205 Capillary tube
1206 Hole
1208 Coextrusion opening
1210 Joining path
1302 dorsoventral temperature gradient
1304 dorsoventral temperature gradient
1306 central, radial temperature gradient
1308 central, radial temperature gradient
1500 photo of cross-sectional fiber profile
1602-1618 cross-sectional fiber profiles
L1: Height of the helical fiber
L2: Length of the non-helical fiber
L3: Height of the infill layer

## Claims

1. An artificial turf fiber (204, 502-510, 804, 902-906, 1500, 1602-1618) comprising a longitudinal cross-sectional profile, the artificial turf fiber being under internal stress that causes the artificial turf fiber, upon receiving heat treatment, to assume a helical shape, wherein the core of the fiber consists of or predominantly comprises a core polymer, and wherein the periphery of the fiber consists of or predominantly comprises a cladding polymer, wherein the core polymer and the cladding polymer are different polymer materials, and wherein the core and cladding polymers are selected such that the core polymer undergoes greater shrinkage than the cladding polymer when subjected to the heat treatment.

2. A helical artificial turf fiber (204, 502-510, 804, 902-906, 1500, 1602-1618) comprising a longitudinal cross-sectional profile, wherein the core of the fiber consists of or predominantly comprises a core polymer, and wherein the periphery of the fiber consists of or predominantly comprises a cladding polymer, wherein the core polymer and the cladding polymer are different polymer materials, and wherein the core and cladding polymers are selected such that the core polymer undergoes greater shrinkage than the cladding polymer when subjected to the heat treatment.

3. The artificial turf fiber according to claim 1 or 2, wherein the cross-sectional profile has a maximum length to maximum thickness ratio of at least 3.0, in particular at least 4.0, in particular at least 5.0,

4. The artificial turf fiber according to any one of the previous claims, wherein the cross-sectional fiber profile has a central bulb and two wings extending from the central bulb in different directions.

5. The artificial turf fiber according to claim 4, wherein the core polymer is comprised in the core of the central bulb and wherein the cladding polymer is comprised in the wings and optionally also in peripheral regions of the central bulb.

6. The artificial turf fiber of any one of the previous claims, wherein the helical artificial turf fiber has a 3D-helical shape **characterized in that** the fiber winds around a central cylinder, wherein the fiber maintains a distance from the cylinder axis as it rotates, and advances along the axis, resulting in a three-dimensional spiral curve.

7. The artificial turf fiber of any one of the preceding claims, wherein the helical artificial turf fiber is a twisted fiber that is rotated around its own axis.

8. The artificial turf fiber according to any one of the claims 1-7, wherein the cross-sectional profile has a maximum length to maximum thickness ratio of at least 3.0, in particular at least 4.0, in particular at least 5.0, and wherein the helical artificial turf fiber is a twisted fiber that is rotated around its own axis.

9. The artificial turf fiber according to any one of the preceding claims, wherein the artificial turf has a core-cladding structure as the result of a co-extrusion step of a stream of the core polymer concentrically surrounded by a stream of the cladding-polymer.

10. The artificial turf fiber according to any one of the preceding claims, wherein the core polymer is or predominantly comprises polyethylene and the cladding polymer is or predominantly comprises polyamide.

11. The artificial turf fiber according to any one of claims, wherein the core polymer is a blend of aged polymer and a first virgin polymer, wherein the cladding polymer is made of a second virgin polymer, and wherein in particular the first and the second virgin polymers are both polyethylene polymers.

12. The artificial turf fiber according to any one of the previous claims, wherein the cladding polymer comprises a higher amount of HDPE than the core polymer.

13. The artificial turf fiber according to any one of the preceding claims, wherein the artificial turf comprises an anti-splicing agent, wherein the anti-splicing agent is in particular a compatibilizer, and/or a low-density polyethylene - LDPE.

14. The artificial turf fiber according to any one of the preceding claims, wherein the fiber comprises a hydrophilization agent, in particular hydrophilic fumed silica, and/or a nucleating agent.

15. An artificial turf, comprising:
- a carrier
- a plurality of helical artificial turf fibers according to any one of the previous claims 2-14, wherein the fibers are integrated into the carrier and extend to one side of the carrier.

16. The artificial turf according to claim 15, further comprising:
- an infill layer,
- wherein the number of twists per fiber and the height of the infill is chosen such that in at least 80% of the helical fibers, the part of the helical fibers that protrudes above the infill layer makes at least 1.0 turns.

17. A method for manufacturing an artificial turf fiber that is under internal stress, the method comprising:
- extruding (104) a combination of at least a core polymer and a cladding polymer into a monofilament via an extrusion nozzle into a monofilament, the monofilament having a longitudinal cross-sectional profile, in particular a cross-sectional profile with a central bulb and two wings extending from the central bulb in different directions, wherein the core and cladding polymers are selected such that the core polymer undergoes greater shrinkage than the cladding polymer when subjected to the heat treatment, wherein the extrusion is performed such that the core polymer is exclusively or predominantly located in the core of the cross-sectional fiber profile and such that the cladding polymer is exclusively or predominantly located in peripheral regions of the cross-sectional profile;
- quenching (106) the monofilament;
- controlling (108) equipment for processing the monofilament such that a temperature gradient is formed reproducibly in the cross-section of the monofilament;
- stretching (110) the monofilament while the temperature gradient is present to form the monofilament into the artificial turf fiber, thereby introducing the internal stress into the fiber, wherein the internal stress is adapted to cause the artificial turf fiber, upon receiving heat treatment, to assume a helical shape.

18. A method for manufacturing a helical artificial turf fiber, comprising:
- Executing the method of claim 17; and
- heating (112) the monofilament, thereby causing the artificial turf fiber to assume a helical shape and become a helical artificial turf fiber.

19. The method according to claims 17 or 18, the method further comprising: creating (102) a polymer mixture comprising the core polymer and the cladding polymer, wherein the polymer mixture is extruded into the monofilament, wherein in particular the extruding is performed via an extrusion channel having a capillary length of less than 2.5 mm, in particular a capillary length of less than 2.0 mm, in particular of 1.4 to 1.6 mm.

20. The method according to claims 17 or 18, wherein the extrusion nozzle is a coextrusion nozzle for co-extruding the core polymer and the cladding polymer such that the core of the fiber is made of the core polymer and that the periphery of the fiber is made of the cladding polymer.

21. The method according to claims 17-20, wherein the method comprises:
- generating a three-phase polymer mixture, the polymer mixture comprising mainly a first polymer, in particular PA, comprising a second polymer, in particular PE, and a compatibilizer, wherein the second polymer forms beads within the first polymer, the beads being surrounded by the compatibilizer, wherein the three phase polymer mixture is used as the extruded at least one mixture; and
- performing the extrusion, wherein the polymer beads are located predominantly in the core o during extrusion, wherein the polymer beads are deformed into thread-like regions in the extrusion process, wherein the thread-like regions of the polymer are located mainly in the core, and wherein the core undergoes greater shrinkage upon being subject to heat-treatment than the periphery of the cross-sectional profile.

22. The method of any one of the previous claims 18-21,
- wherein the cross-sectional profile has a maximum length to maximum thickness ratio of below 3.0, and wherein the equipment is controlled such that a dorso-ventral temperature gradient is formed; or
- wherein the cross-sectional profile has a maximum length to maximum thickness ratio of at least 3.0, in particular at least 4.0, in particular at least 5.0, and wherein the equipment is controlled such that a radial temperature gradient is formed.
